# EUROPEAN PATENT APPLICATION

(11) **EP 0 812 912 A1**
(43) Date of publication of application: **17.12.1997**
(21) Application number: 97107205.3
(22) Date of filing: 20.09.1989
(51) Int. Cl.: C12N 15/11, C12N 9/00

(54) **RNA Catalyst for cleaving specific RNA sequences**

(30) Priority: 20.09.1988 US 247100
(62) Divisional of application: 95115981.3
(71) Applicant: THE BOARD OF REGENTS FOR NORTHERN ILLINOIS UNIVERSITY, DeKalb, Illinois 60115 (US); BIOTECHNOLOGY RESEARCH AND DEVELOPMENT CORPORATION INCORPORATED, Peoria Illinois 61604 (US)
(72) Inventor: Hampel, Arnold E., Dekalb, Illinois 60115 (US); Tritz, Richard H., Apt. E9, Dekalb, Illinois 60115 (US); Hicks, Margaret F., Antioch, Tennessee 37013 (US)
(74) Representative: Grünecker, Kinkeldey, Stockmair & Schwanhäusser Anwaltssozietät

(57) **Abstract**

A synthetic RNA catalyst capable of cleaving an RNA substrate, the catalyst comprising a substrate binding portion and a "hairpin" portion. The invention also provides an engineered DNA molecule and a vector, each comprising a DNA sequence coding for an RNA catalyst according to the invention. The invention further comprises host cells transformed with the vectors of the invention which are capable of expressing the RNA substrate. Finally, the invention provides a method of cleaving an RNA substrate which comprises contacting the substrate with a synthetic RNA catalyst according to the invention.

## Description

This application is a continuation-in-part of application Serial No. 07/247,100 filed September 20, 1988, the disclosure of which is incorporated herein by reference.

### FIELD OF THE INVENTION

The present invention relates to an RNA catalyst which cleaves specific RNA sequences into a fragment having a 5' hydroxyl and a fragment having a 2',3' cyclic phosphate. The products of the reaction described herein resemble those resulting from the natural hydrolysis of RNA.

### BACKGROUND OF THE INVENTION

Certain naturally occurring satellite, virusoid and viroid RNAs possess the property of self-catalyzed cleavage. Self-cleavage has been demonstrated in vitro for avocado sunblotch viroid (ASBV) (Hutchins, C. J., Rathjen, P. D., Forster, A. C. and Symons, R. H. (1986) Nucleic Acids Res., 14: 3627-3640), satellite RNA from tobacco ringspot virus (sTRSV) (Prody, G. A., Bakos, J. T., Buzayan, J. M., Schneider, I. R. and Bruening, G. (1986) Science, 231: 1577-1580; Buzayan, J. M., Gerlach, W. L. and Bruening, G. B. (1986) Proc. Natl. Acad. Sci. U.S.A. 83: 8859-8862) and lucerne transient streak virus (vLTSV) (Forster, A. C. and Symons, R. H. (1987) Cell, 49:
211-220). These self-catalyzed RNA cleavage reactions share a requirement for divalent metal ions and neutral or higher pH and cleave target RNA sequences to give 5' hydroxyl and 2',3'-cyclic phosphate termini (Prody, G. A., Bakos, J. T., Buzayan, J. M., Schneider, I. R. and Bruening, G. (1986) Science, 231: 1577-1580; Forster, A. C. and Symons, R. H. (1987) Cell, 49: 211-220; Epstein, L. M. and Gall, J. G. (1987) Cell, 48: 535-543; Buzayan, J. M. Gerlach, W. L., Bruening, G. B., Keese, P. and Gould, A. R. (1986) Virology, 151: 186-199).

A "hammerhead" model has been proposed and accurately describes the catalytic center of (+)sTRSV RNA, the (+) and (-) strands of ASBV and the (+) and (-) strands of vLTSV (Forster, A. C. and Symons, R. H. (1987) Cell, 49: 211-220). The single exception is (-)sTRSV RNA which does not fit the "hammerhead" model (Forster, A. C. and Symons, R. H. (1987) Cell, 49: 211-220; Buzayan, J. M., Gerlach, W. L. and Bruening, G. (1986) Nature, 323: 349-352; Buzayan, J. M., Hampel, A. and Bruening, G. B. (1986) Nucleic Acids Res., 14: 9729-9743), and the structure of whose catalytic center was unknown prior to the present invention. It is therefore understandable that the primary scientific focus has been on studying the "hammerhead" consensus structure and, as regards sTRSV, on studying the (+) strand.

Intermolecular cleavage of an RNA substrate by an RNA catalyst that fits the "hammerhead" model was first shown in 1987 (Uhlenbeck, O. C. (1987) Nature, 328: 596-600). The RNA catalyst was recovered and reacted with multiple RNA molecules, demonstrating that it was truly catalytic.

Catalytic RNAs designed based on the "hammerhead" motif has been used to cleave specific target sequences by making appropriate base changes in the catalytic RNA to maintain necessary base pairing with the target sequences (Haseloff and Gerlach, Nature, 334, 585 (1988); Walbot and Bruening, Nature, 334, 196 (1988); Uhlenbeck, O. C. (1987) Nature, 328: 596-600; Koizumi, M., Iwai, S. and Ohtsuka, E. (1988) FEBS Lett., 228: 228-230). This has allowed use of the catalytic RNA to cleave specific target sequences and indicates that catalytic RNAs designed according to the "hammerhead" model may possibly cleave specific substrate RNAs in vivo. (see Haseloff and Gerlach, Nature, 334, 585 (1988); Walbot and Bruening, Nature, 334, 196 (1988); Uhlenbeck, O. C. (1987) Nature, 328: 596-600).

However, catalytic RNAs such as these that were designed based on the "hammerhead" model have several limitations which restrict their use in vitro and may forestall their use in vivo. For example, the temperature optimum for the reaction is 50-55°C, which is well above physiological, and the kcat (turnover number) is only 0.5/min even at 55°C (Uhlenbeck, O. C. (1987) Nature, 328:596-600; Haseloff and Gerlach, Nature, 334, 585 (1988)). In addition, the Km is 0.6uM (Uhlenbeck, O. C. (1987) Nature, 328:596-600) meaning that the reaction requires high concentrations of substrate which makes it difficult, if not impossible, for the catalytic RNA to cleave low levels of target RNA substrate such as would be encountered in vivo.

Cech et al. published application WO 88/04300 also reports the preparation and use of certain synthetic ribozymes that have several activities, including endoribonuclease activity. The design of these ribozymes is based on the properties of the Tetrahymena ribosomal RNA self-splicing reaction. A temperature optimum of 50^{o}C is reported (pages 37 and 39) for the endoribonuclease actvity, and the Km and kcat reported for this activity were 0.8uM and 0.13/minute, respectively (page 44).

In view of the above, there is a need for an RNA catalyst having a lower temperature optimum, preferably near physiological temperatures, a higher turn-over number and a smaller Km and which can be engineered to cut specific target RNA substrates Accordingly, based on the discovery of a totally different structure disclosed hereinafter, it is an object of the present invention to provide such an RNA catalyst. Other objects and features of the invention will be in part apparent and in part pointed out. The invention accordingly comprises the products and methods hereinafter described and their equivalents, the scope of the invention being indicated in the appended claims.

### SUMMARY OF THE INVENTION

The invention comprises a synthetic RNA catalyst capable of cleaving an RNA substrate which contains the sequence:
5'-F₁-CS-F₂-3',
wherein,
CS is a cleavage sequence; and
F₁ and F₂ each is a sequence of bases flanking the cleavage sequence.

The catalyst comprises a substrate binding portion and a "hairpin" portion. The substrate binding portion of the catalyst has the sequence:
3'-F₄-L₁-F₃-5'
wherein,
F₃ is a sequence of bases selected so that F₃ is substantially base paired with F₂ when the catalyst is bound to the substrate;
F₄ is a sequence of bases selected so that F₄ is substantially base paired with F₁ when the catalyst is bound to the substrate;
the sequences of F₃ and F₄ being selected so that each contains an adequate number of bases to achieve sufficient binding of the RNA substrate to the RNA catalyst so that cleavage of the substrate can take place; and
L₁ is a sequence of bases selected so that L₁ does not base pair with CS when the catalyst is bound to the substrate.

The "hairpin" portion is a portion of the catalyst that assumes a hairpin-like configuration when the substrate-catalyst complex is modeled in two dimensions for minimum energy folding. The "hairpin" portion of the catalyst preferably has the sequence: 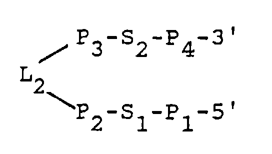 wherein,
P₁ and P₄ each is a sequence of bases, the sequences of P₁ and P₄ being selected so that P₁ and P₄ are substantially base paired;
P₁ is covalently attached to F₄,
S₁ and S₂ each is a sequence of bases, the sequences of S₁ and S₂ being selected so that S₁ and S₂ are substantially unpaired;
P₂ and P₃ each is a sequence of bases, the sequences of P₂ and P₃ being selected so that P₂ and P₃ are substantially base paired; and
L₂ is a sequence of unpaired bases.

RNA catalysts according to the invention can cleave substrates of any length or type as long as they contain an appropriate cleavage sequence. In particular, the catalysts can be used to cleave a specific sequence in naturally-occuring RNA having a cleavage sequence, as well as RNAs which have been engineered to contain a cleavage sequence.

The invention further comprises an engineered DNA molecule and a vector, each of which comprises a DNA sequence that codes for an RNA catalyst according to the invention. The invention also comprises a host transformed with the vector, the host being capable of expressing the RNA catalyst. In particular, hosts can be transformed with vectors that, when transcribed, will produce RNA catalysts which can cleave any RNA, native or foreign, found in the host. For example, hosts can be transformed with vectors that, when transcribed, produce RNA catalysts which can regulate the expression of genes by cleaving messenger RNA or which act as anti-viral agents by cleaving viral RNA. Thus, the invention has application in vitro and in vivo in prokaryotes and eukaryotes of plant or animal origin in regulating gene expression and for controlling viral infections.

Finally, the invention includes a method of cleaving an RNA substrate comprising contacting the substrate with an RNA catalyst according to the invention. The reaction is unique because it occurs under physiological conditions, having a temperature optimum near 37°C, with very favorable reaction parameters. The method can be practiced in vitro or in vivo. For instance, the method may be practiced in vivo in host cells that have been transformed with a vector that codes for an RNA catalyst according to the invention.

### BRIEF DESCRIPTION OF THE DRAWINGS

Figure 1 shows the (-)sTRSV RNA substrate-catalyst complex that fits the "hairpin" model of catalytic RNA in accordance with the present invention.

Figure 2 shows minimum energy folding of (-)sTRSV RNA.

Figure 3 shows the time course of catalysis of a substrate RNA by the catalytic RNA.

Figure 4 shows the Michaelis-Menten kinetics of the RNA catalytic reaction.

Figure 5 shows the temperature dependence of the RNA catalytic reaction.

Figure 6 shows the dependence of the rate of reaction on concentration of catalytic RNA.

Figure 7 shows the reaction properties of a smaller RNA substrate.

Figure 8 shows loss of catalytic activity when the terminal A at position 175 or the terminal bases AU at positions 175 and 176 are removed from the catalytic RNA.

Figure 9 shows loss of catalytic activity when bases 195-203 in the catalytic RNA sequence are removed.

Figure 10 shows loss of catalytic activity when bases AAA at positions 203, 202 and 201 are changed to CGU respectively.

Figures 11A-C show that there is no effect on catalytic activity when base A at position 49 in the substrate is changed to a G, U or C.

Figure 12 shows that different target RNA sequences can be used as long as the base pairing with the catalytic RNA in the regions flanking the cleavage sequence is maintained.

Figure 13 shows that an RNA sequence found in tobacco mosaic virus can be cleaved at a specific site with the catalytic RNA of the present invention.

Figures 14A-C show three substrates having sequences found in the sequence of the messenger RNA coding for chloramphenicol acetyl transferase. Figures 14A-C also show the separation patterns on acrylamide gels of the reaction products obtained by reacting these substrates with catalytic RNAs designed to base pair with the substrates in the regions flanking the AGUC cleavage sequence.

Figure 15 shows the sequence of a substrate having a sequence found in the sequence coding for the gag protein of the HIV-1 virus which causes AIDS. Figure 15 also shows the separation patterns on acrylamide gels of the reaction products obtained by reacting this substrate with a catalytic RNA designed to base pair with the substrate in the regions flanking the CGUC cleavage sequence of the substrate.

Figure 16 shows the sequence of a substrate having a sequence found in the sequence coding for the regulatory tat protein of the HIV-1 virus. Figure 16 also shows the separation patterns on an acrylamide gel of the reaction products obtained by reacting this substrate with a catalytic RNA designed to base pair with the substrate in the regions flanking the UGUC cleavage sequence of the substrate.

Figure 17 shows the sequence of a substrate having four non-native U's added to the 3' end of the sequence of the native (-)sTRSV substrate shown in Figure 1. Figure 17 also shows the separation patterns on an acrylamide gel of the reaction products obtained by reacting this substrate with different concentrations of a catalytic RNA designed to base pair with the substrate in the regions flanking the cleavage sequence of the substrate, including with the four non-native U's.

Figure 18 summarizes the sequence requirements for the target region of the substrate RNA. Only GUC is required for cleavage.

Figure 19 shows the positions of base changes (open boxes) made in the sequence of the catalytic RNA shown in Figure 1 in order to prove the existence of Helices 3 and 4 predicted by the "hairpin" model for (-)sTRSV RNA. Figure 19 also shows the separation patterns on acrylamide gels of the reaction products obtained by reacting the various catalytic RNAs with substrate RNA S17.

Figure 20 shows the RNA sequence of an autocatalytic cassette that has utility in terminating transcription at a very specific site. Figure 20 also shows the separation pattern on an acrylamide gel of the reaction products obtained when this catalyst was transcribed and cleaved autocatalytically.

Figure 21 shows the positions of two base changes that were made in the native (-)sTRSV catalytic RNA sequence shown in Figure 1. Figure 21 also shows the separation patterns on acrylamide gels of the reaction products obtained by reacting these catalysts or catalyst R51 (control gel) with substrate S17.

Figure 22A shows a partial map of plasmid pHC-CAT containing the CAT gene linked to the "hairpin" autocatalytic cassette of the invention. Also shown is the expected RNA transcript of the illustrated region and the expected 5' fragment of the autocatalytic cleavage.

Figure 22B shows the result of Northern blot analysis of RNA produced by host cells transformed with pHC-CAT.

### DETAILED DESCRIPTION OF THE DRAWINGS

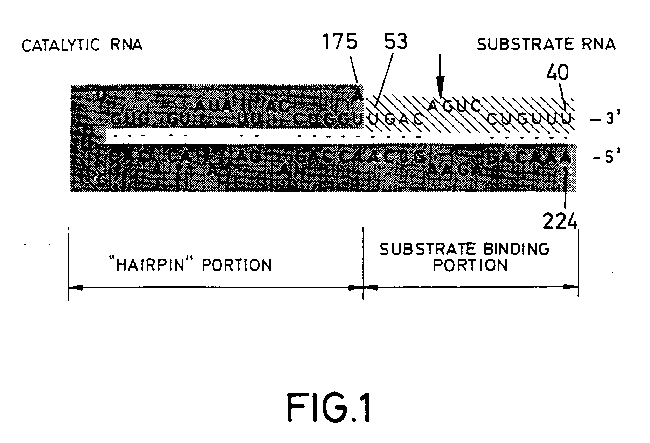

Figure 1. The "hairpin" model for RNA catalysis was developed by determining the minimum energy folding predicted by computer modeling of the catalytic complex containing the minimum size catalytic RNA and substrate RNA of (-)sTRSV RNA. It is this minimum energy folding which is shown in Figure 1. Two molecules are shown folded: (1) catalytic RNA which contains 50 bases of satellite RNA (224-175) and (2) substrate RNA which contains 14 bases of satellite RNA (53-40). The arrow represents the site of cleavage.

The 50-base catalytic RNA and the 14-base substrate RNA are the "minimum size" in the sense that reductions in their length result in a substantial or total loss of catalytic activity as is shown in the Examples below. Thus, this length of (-)sTRSV catalyst sequence is preferred to shorter lengths. Also, substrate RNA having at least the degree of base-pairing with the catalyst exhibited by the 14-base substrate is preferred.

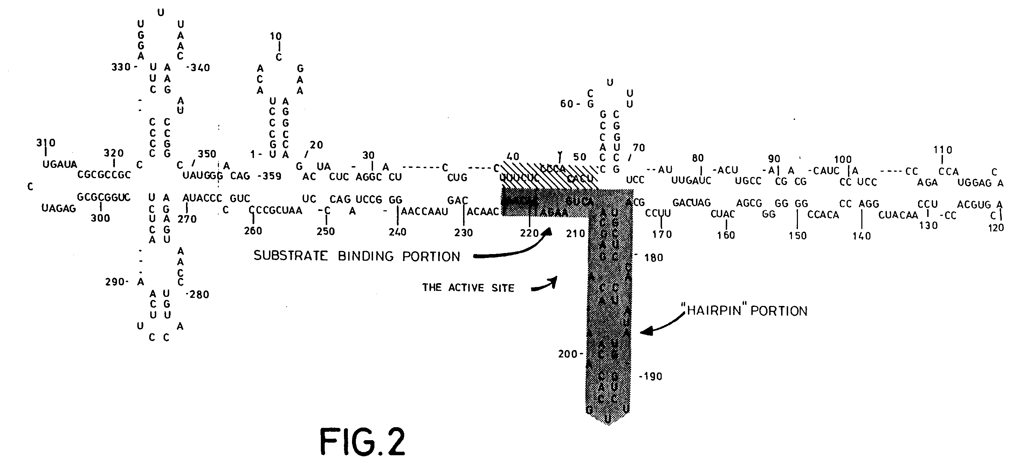

Figure 2. Minimum energy folding of (-)sTRSV RNA. The molecule was folded using the Wisconsin RNA folding program (Zucker, M. and Stiegler, P. (1981) Nucleic Acids Res., 9: 133-148; Devereux, J., Haeberli, P. and Smithies, O. (1984) Nucleic Acids Res., 12: 387-395) with base numbers corresponding to (+)sTRSV (Buzayan, J. M., Gerlach, W. L., Bruening, G. B., Keese, P. and Gould, A. R. (1986) Virology, 151: 186-199). With this numbering scheme the 5'-3' direction of the molecule is with decreasing base number. The minimum catalytic complex is identified. The substrate RNA sequence is between bases 53-40 and the catalytic RNA sequence is between bases 224-175. The arrow is the site of cleavage.

The folding identifies regions of expected base pairing and expected non-base pairing, loops. This model does not preclude higher order interactions occurring between the loops.

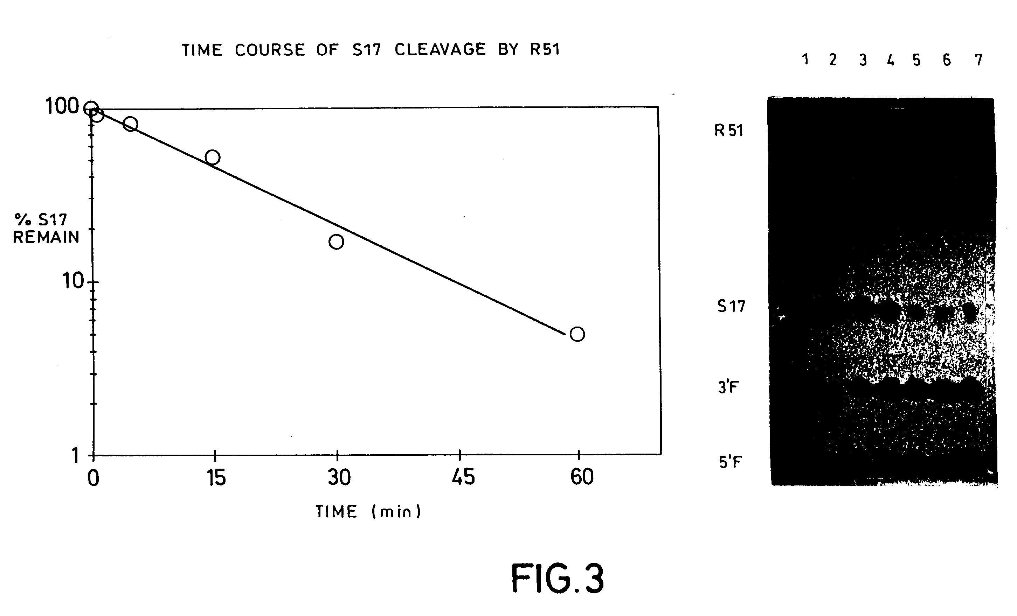

Figure 3. Time course of substrate S17 cleavage by catalytic RNA R51. The reaction was carried out under standard conditions, which were 37°C in 12mM MgCl₂, 40mM Tris pH 7.5 and 2mM spermidine, for the following times: lane 1, 30 sec; lane 2, 5 min; lane 3, 15 min; lane 4, 30 min; lane 5, 60 min; lane 6, 90 min; lane 7, 150 min. Concentrations were as follows: [R51]=0.0032uM and [S17]=0.09uM. RNA was separated on 7M urea, 20% acrylamide gels, bands cut out and counted in the liquid scintillation counter for Figures 3-17 and 19-21. Throughout the figures, the designations 5'F and 3'F are the products of cleavage of the substrate and represent the resulting 5' fragments and 3' fragments, respectively.

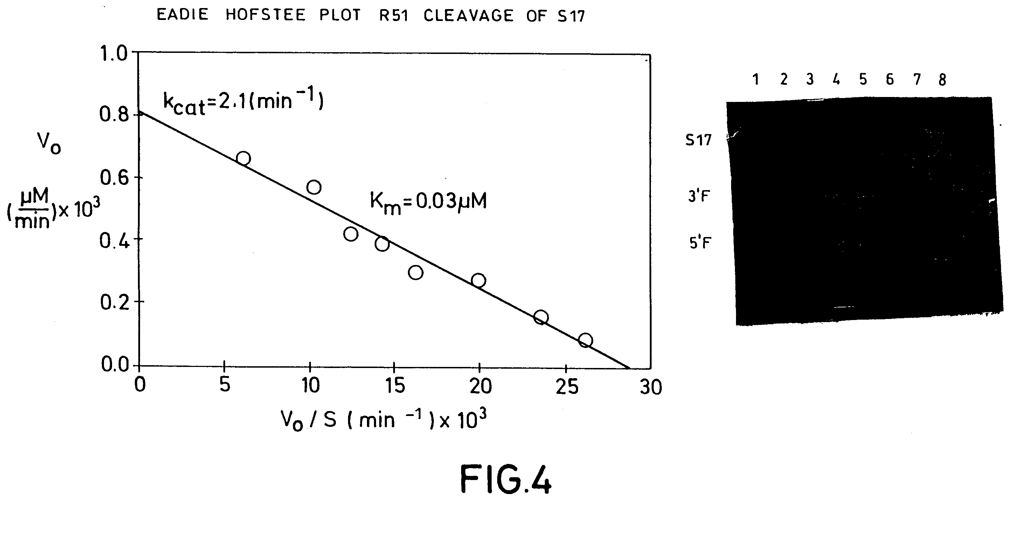

Figure 4. Eadie Hofstee plot of catalytic RNA R51 cleavage of substrate RNA S17. The reaction was carried out at 37°C in 12mM MgCl₂, 40mM Tris pH 7.5 and 2mM spermidine. Concentrations were as follows: [R51]=0.0004uM and [S17]=0.125uM (lane 1), 0.0625uM (lane 2), 0.0417uM (lane 3), 0.031uM (lane 4), 0.021uM (lane 5), 0.0156uM (lane 6), 0.0078uM (lane 7) and 0.0039uM (lane 8).

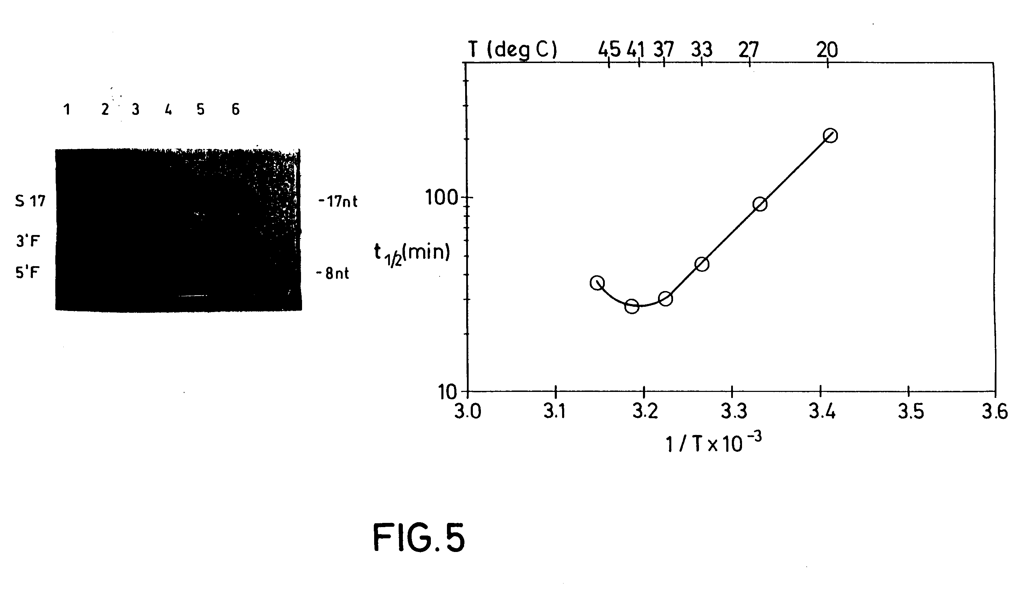

Figure 5. Temperature dependence of the rate of cleavage of substrate RNA S17 by catalytic RNA R51. The reaction was carried out in 12mM MgCl₂, 40mM Tris pH 7.5 and 2mM spermidine at 45^{o}C (lane 6), 41°C (lane 5), 37°C (lane 4), 33°C (lane 3), 27°C (lane 2) and 20°C (lane 1). The concentrations used were: [R51]= 0.003uM and [S17]=0.09uM. R51 was unlabeled. The velocities shown in the graph in Figure 5 were calculated by the use of time points of 8 and 16 minutes. The separation patterns of the reaction products on the acrylamide gel shown in the figure are for the 16-minute time point.

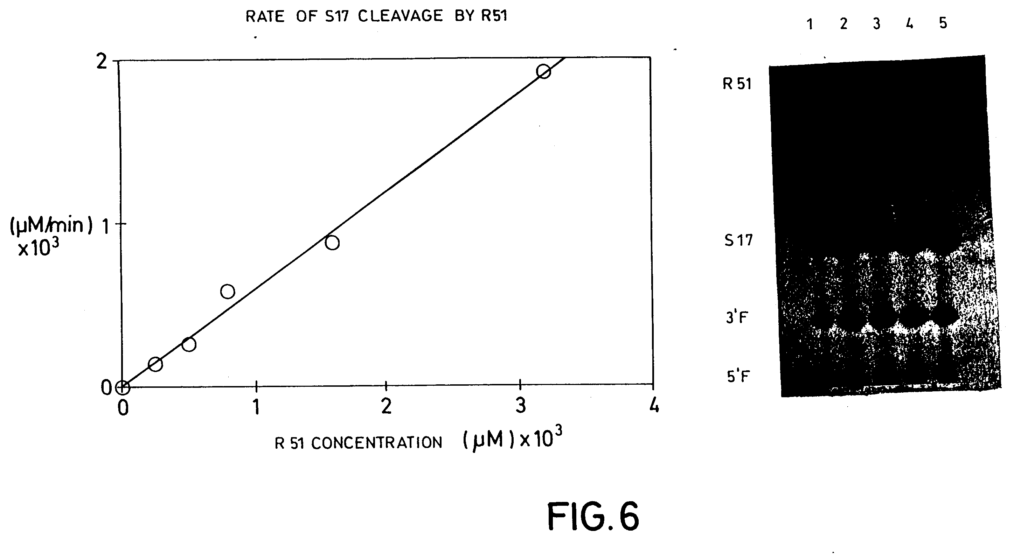

Figure 6. Rate of cleavage of substrate RNA S17 at varying concentrations of catalytic RNA R51. The reaction was carried out at 37°C in 12mM MgCl₂, 40mM Tris pH 7.5 and 2mM spermidine for 40 min (lane 1 and 2), 20 min (lane 3), 10 min (lane 4) and 5 min (lane 5). The concentration of substrate used was 0.175uM.

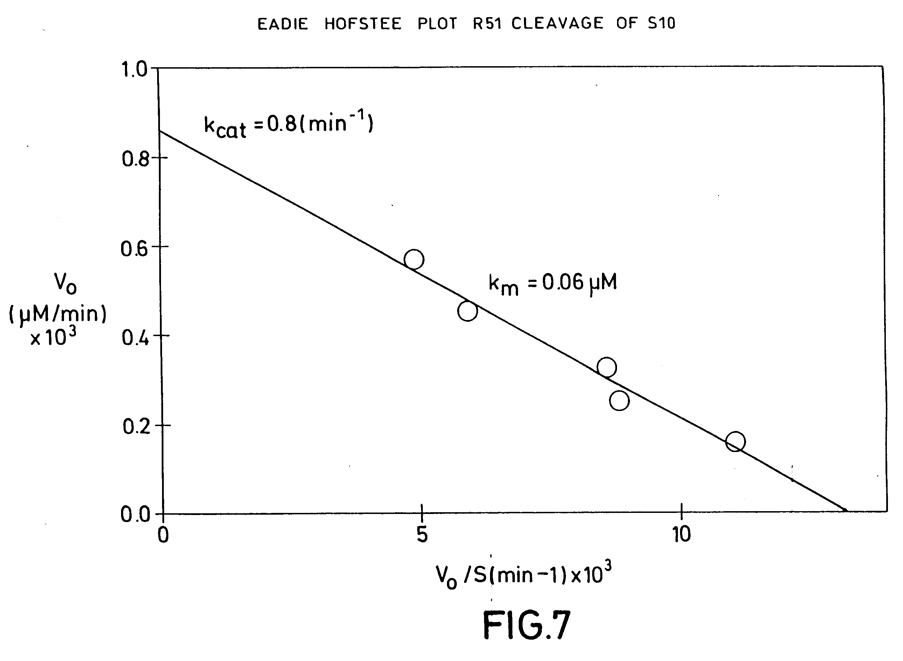

Figure 7. Eadie Hofstee plot of catalytic RNA R51 cleavage of substrate RNA S10. The substrate S-10 containing the RNA sequence: GACAGUCCUG was prepared from a DNA template containing the T-7 promoter as described in Example 2. This substrate was mixed with the catalytic RNA, R51, from Example 2 under standard conditions: 37°C in 12mM MgCl₂, 40mM Tris pH 7.5 and 2mM spermidine for 10 min. Concentrations of substrate used were as follows: 0.115uM, 077uM, 0.038uM, 0.029uM, 0.014uM. The concentration of catalytic RNA, R51, used was 1nM. The line was fit by linear regression analysis and intercept, kcat, and Michaelis constant, Km, calculated.

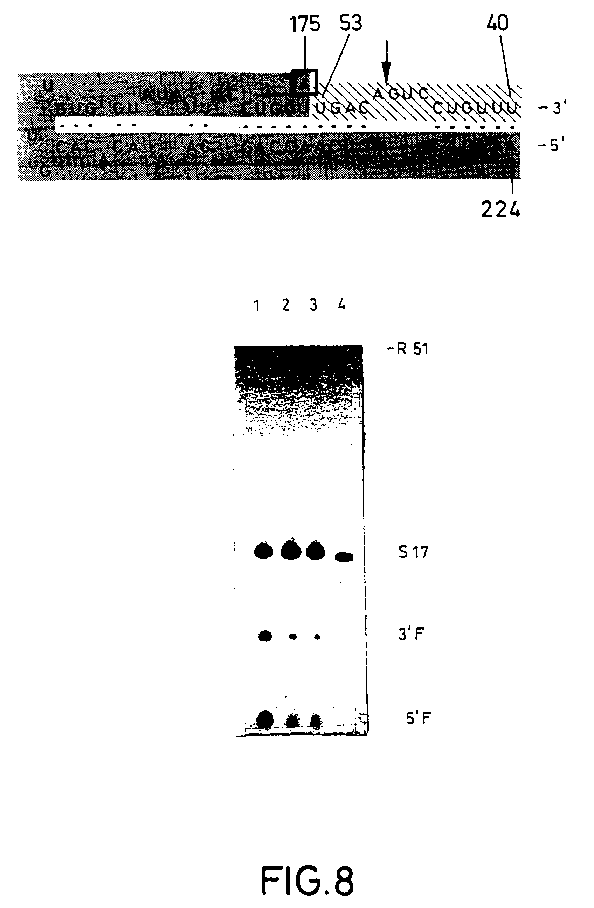

Figure 8. Removal of the terminal "A" at position 175 of the catalytic RNA. The "A" at base position 175 (circled) was removed and the resulting catalytic RNA, R50, reacted with substrate S17. In addition, two bases were removed, to give R49 which had both A175 and U176 removed. Concentration of substrate S17 was 0.3uM and all catalytic RNA concentrations were 4nM. The reaction times were 20 min under standard conditions. Lane 1 R51/S17; Lane 2 R50/S17; Lane 3 R49/S17; Lane 4 S17 alone. A 75% loss of activity was seen with R50 and R49.

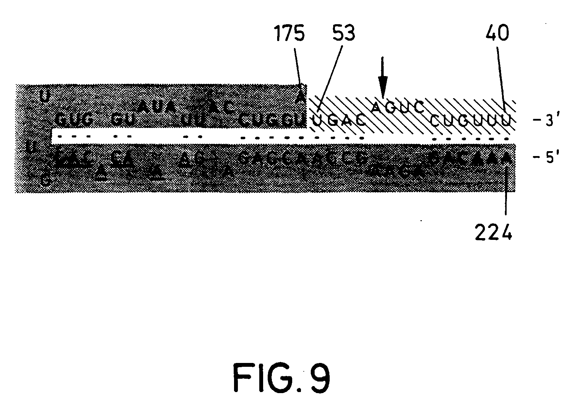

Figure 9. Loss of activity when bases 195-203 in the catalytic RNA sequence are removed. When the underlined bases were removed and the adjacent bases ligated together, no catalytic activity was seen.

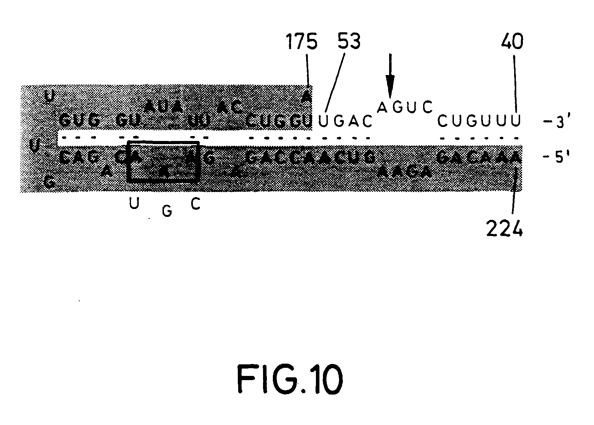

Figure 10. Loss of activity when bases AAA at positions 203, 202 and 201 are changed to CGU respectively. When the circled AAA bases were replaced by the underlying 5'-CGU-3' bases, no catalytic activity was seen.

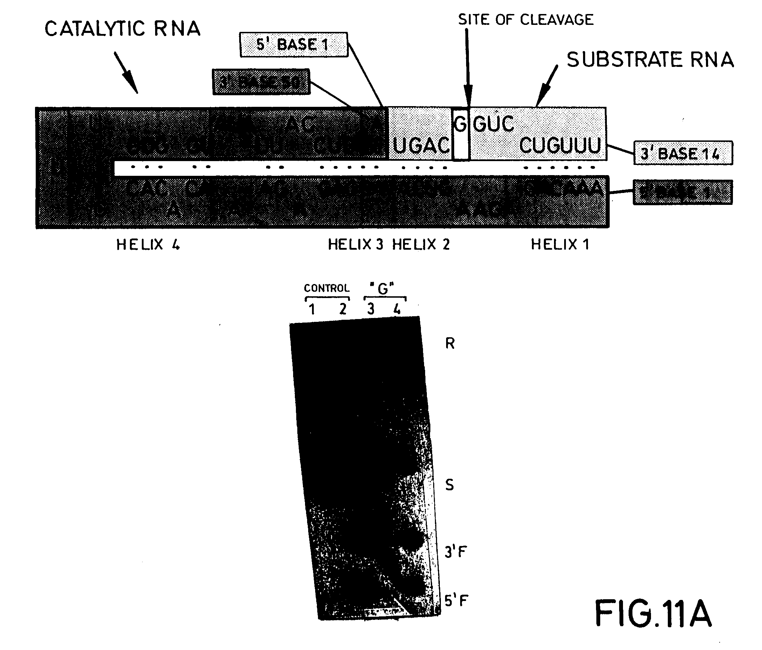

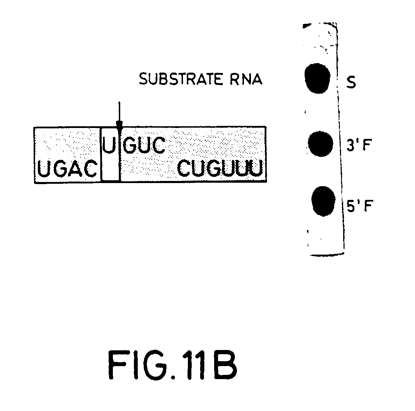

Figure 11A. No effect on activity when base A49 in the substrate is changed to a "G". The circled "A" base 49 in the substrate was changed to a "G" and no effect on activity was seen. The concentration of R51 was 0.016uM, [S17]=0.4uM, and [S17(A->G)]=0.2uM. Reaction under standard conditions was for 40 min. Lane 1 S17; Lane 2 S17/R51; Lane 3 S17(A->G); Lane 4 S17(A->G)/R51.

Figure 11B. No effect on activity was seen when base A49 in the substrate was changed to a "U" (S17(A-->U)). The concentration of substrate RNA S17(A-->U) used was 0.12uM and the concentration of R51 was 0.0065uM. Reaction was for 60 minutes under standard conditions. The catalytic RNA was unlabelled.

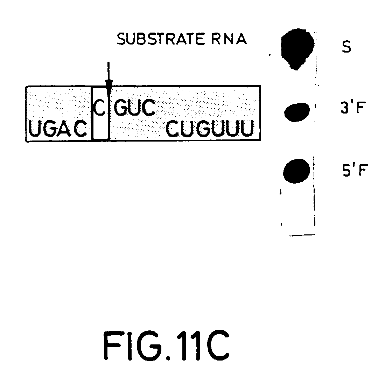

Figure 11C. No effect on activity was seen when base A49 in the substrate was changed to a "C" (S17(A-->C)). The concentration of substrate RNA S17(A-->C) used was 0.08uM and the concentration of R51 was 0.0065uM. Reaction was for 60 minutes under standard conditions. The catalytic RNA was unlabelled.

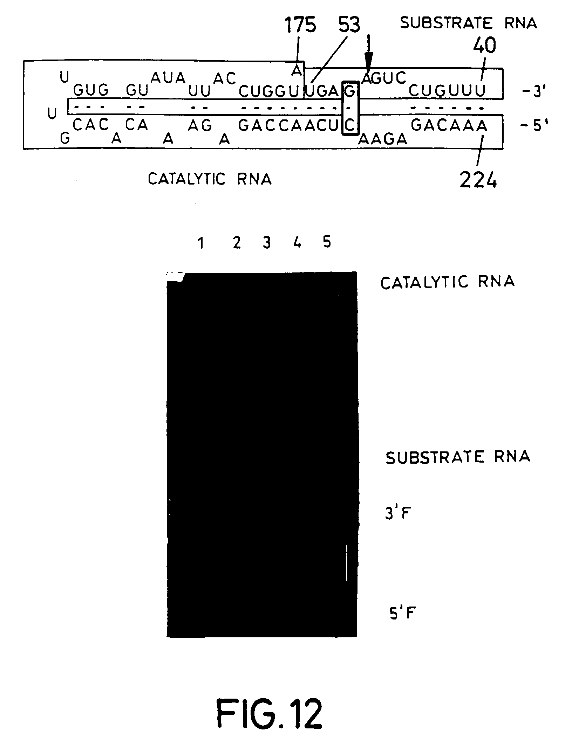

Figure 12. Different target RNA sequences can be used as long as the base pairing is maintained with the catalytic RNA. The C:G base pair predicted by the "hairpin" model of the catalytic complex of (-)sTRSV, Figure 1, was changed to a G:C base pair (circled) and activity was maintained. In this experiment the usual substrate S17 was not used; instead a new substrate was used with the exact same sequence except the first two vector bases GC were eliminated. The resulting sequence of this new substrate S15 was gUGACAGUCCUGUUU. The substrate containing the C50->G50 base change was S15(C->G) and the catalytic RNA with the G214->C214 base change was R51(G->C). The reactions were run under standard conditions for 60 min at [R51]=0.07uM, [S151=0.12uM, [S15(C->G)]=0.15uM, [R51(G->C)]=0.05uM. Lane 1 R51/S15; Lane 2 R51(G->C)/S15; Lane 3 S15; Lane 4 R51(G->C)/S15(C->G); Lane 5 S15(C->G).

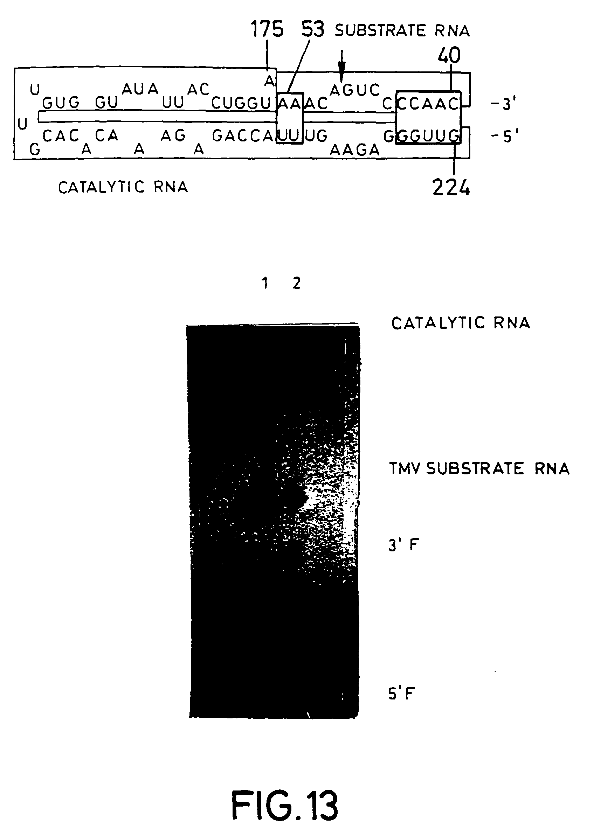

Figure 13. An RNA sequence found in tobacco mosaic virus (TMV) can be cleaved at a specific site. The substrate RNA shown is that beginning with nucleotide #538 in the tobacco mosaic virus sequence. The catalytic RNA has been synthesized to base pair with the TMV substrate RNA in the stem regions of the "hairpin" as shown by the circled base pairs. Reaction was for 20 min under standard conditions with a catalytic RNA concentration of 3nM and a substrate concentration of 0.06uM. Lane 1 TMV substrate RNA only; Lane 2 TMV catalytic RNA/TMV substrate RNA.

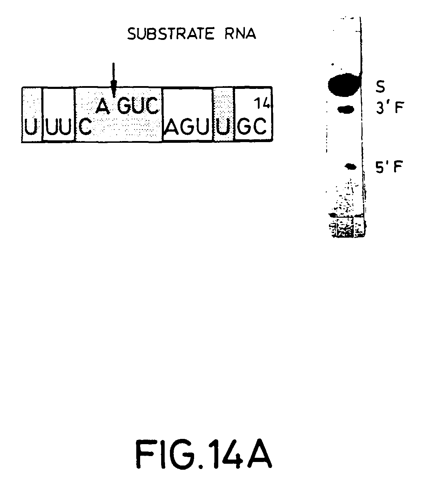

Figures 14A-C. The sequences of three substrate RNAs having sequences found in the messenger RNA for chloramphenicol acetyl transferase (CAT) are shown. They have 14-, 16- and 18-base long target sites, and the length of the 3' regions flanking the AGUC cleavage sequence is extended in substrates (B) and (C) as compared to substrate (A). Catalytic RNAs designed to base pair with the substrate RNAs in both the 3' and 5' regions flanking the cleavage sequence ACUC were synthesized. The open boxed bases are those which are different from those in the native (-)sTRSV substrate RNA sequence shown in Fig. 1.

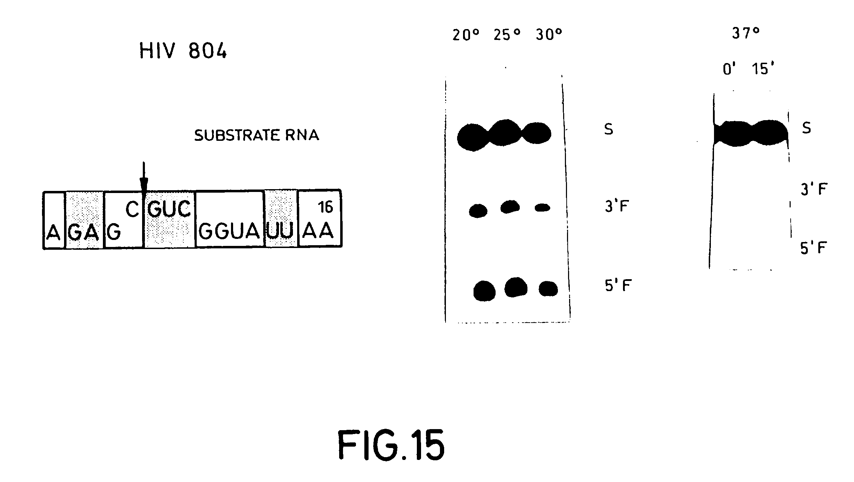

Figure 15. This figure shows the sequence of a substrate RNA having a sequence found in the region of the HIV-1 viral RNA which specifies the gag protein. A catalytic RNA was made whose sequence was designed so that the catalyst would base pair with the substrate RNA in both the 3' and 5' regions flanking the cleavage sequence. The open boxed bases are those which are different than those of the native (-)sTRSV sequence shown in Figure 1. The catalytic RNA cleaved the substrate RNA at the arrow.

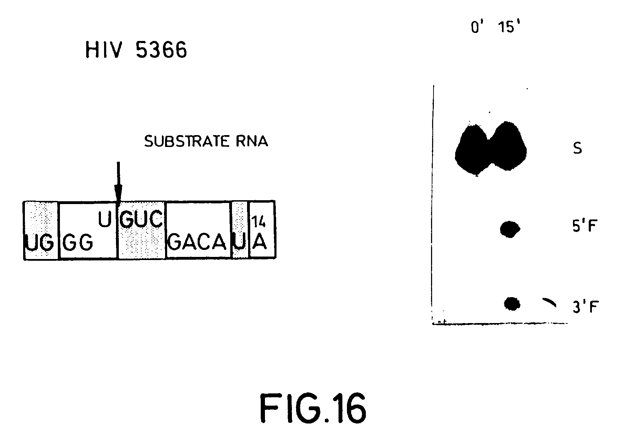

Figure 16. Shown is the sequence of a substrate RNA having the sequence found at the beginning of the coding region for the regulatory protein tat of the HIV-1 virus. A catalytic RNA was made which was designed so that it would base pair with the substrate RNA in both the 3' and 5' regions flanking the UGUC catalytic cleavage sequence. The open boxes are bases which are different from those of the native (-)sTRSV substrate sequence shown in Figure 1. Cleavage occurred at the arrow as shown.

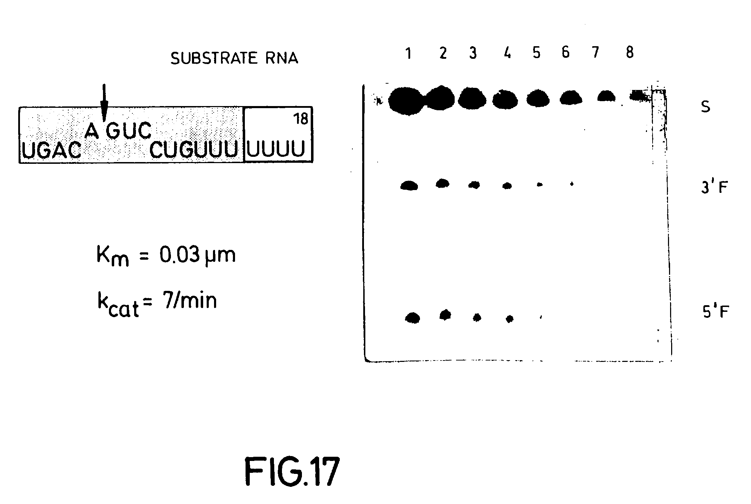

Figure 17. Shown is the sequence of a substrate RNA having four non-native bases (UUUU) added to the 3' end of the sequence of the native (-)sTRSV substrate RNA shown in Fig. 1. A corresponding catalytic RNA was made whose sequence was designed so it would base pair with the substrate in both the 3' and 5' regions flanking the AGUC cleavage sequence. Cleavage rates with a constant catalytic RNA concentration and various concentrations of substrate RNA were determined by cutting out the bands of the acrylamide gels, counting the radioactivity and plotting the data using Michaelis-Menton procedures to calculate Km and kcat.

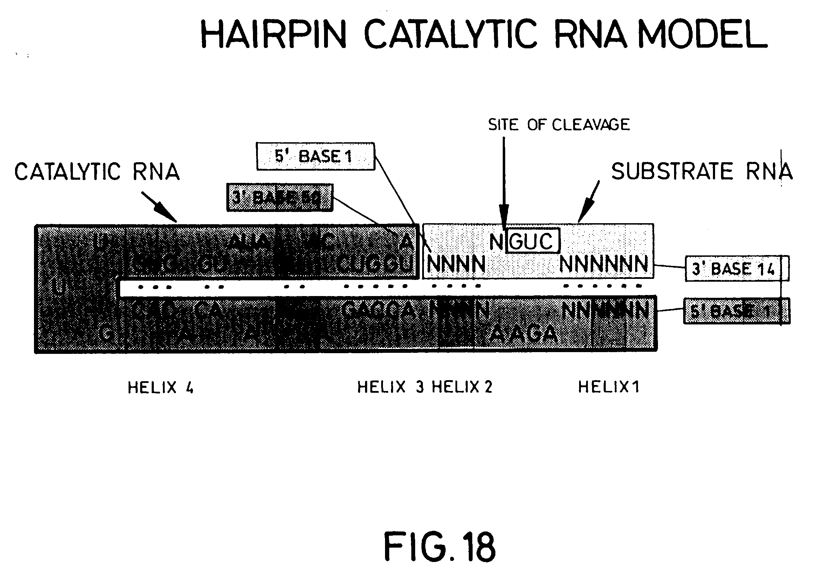

Figure 18. Summary of the sequence requirements for the target region of substrate RNA. Only a GUC sequence is required for cleavage of the substrate as long as the short sequences of bases in the regions of the substrate flanking the cleavage sequence are substantially base paired with corresponding regions of the RNA catalyst. The regions of base pairing are labeled Helix 1 and Helix 2 in the figure. Also, the regions of base pairing predicted by the "hairpin" model for (-)sTRSV to exist in the "hairpin" portion of the catalyst are labeled Helices 3 and 4.

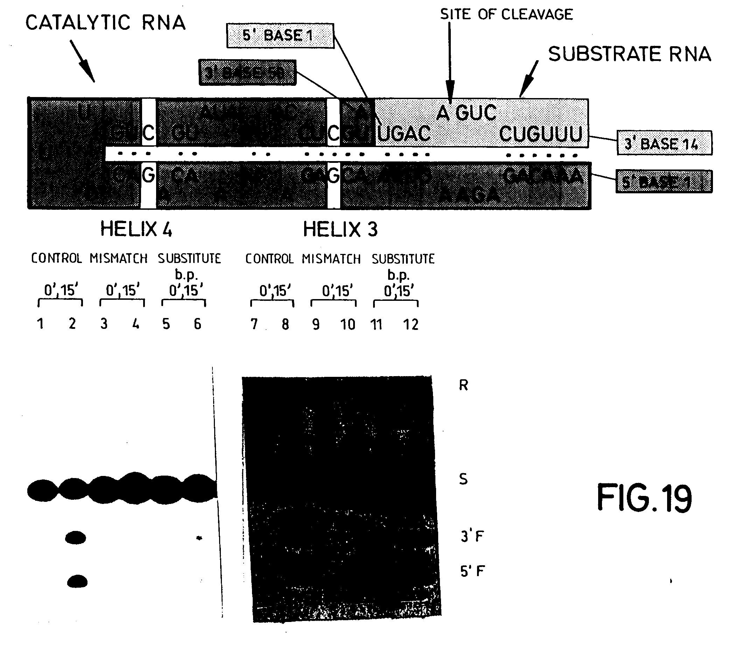

Figure 19. Confirmation of the existence of Helices 3 and 4 predicted by the "hairpin" model for (-)sTRSV RNA. A G->C base mutation in base 35 (count bases from the 5' end of the catalytic RNA sequence shown) of the (-)sTRSV catalytic RNA sequence shown in Fig. 1 resulted in an RNA with no catalytic activity (Lanes 3 and 4 ("mismatch")). A double mutant, G35->C; C27->G had restored catalytic activity (Lanes 5 and 6 ("substitute b.p.")). These two base changes are in the Helix 4 region whose existence is predicted by the "hairpin" model for (-)sTRSV. Also, a catalytic RNA having a single base change at position 47 (G47->C) was inactive (Lanes 9 and 10), while a double mutant, with a second mutation C17->G, had restored activity (Lanes 11 and 12). These two base changes are in the Helix 3 region whose existence is predicted by the "hairpin" model. The control (Lanes 1, 2, 7 and 8) is cleavage of the substrate RNA S17 having the native (-)sTRSV sequence by catalytic RNA sequence R51 having the native sequence.

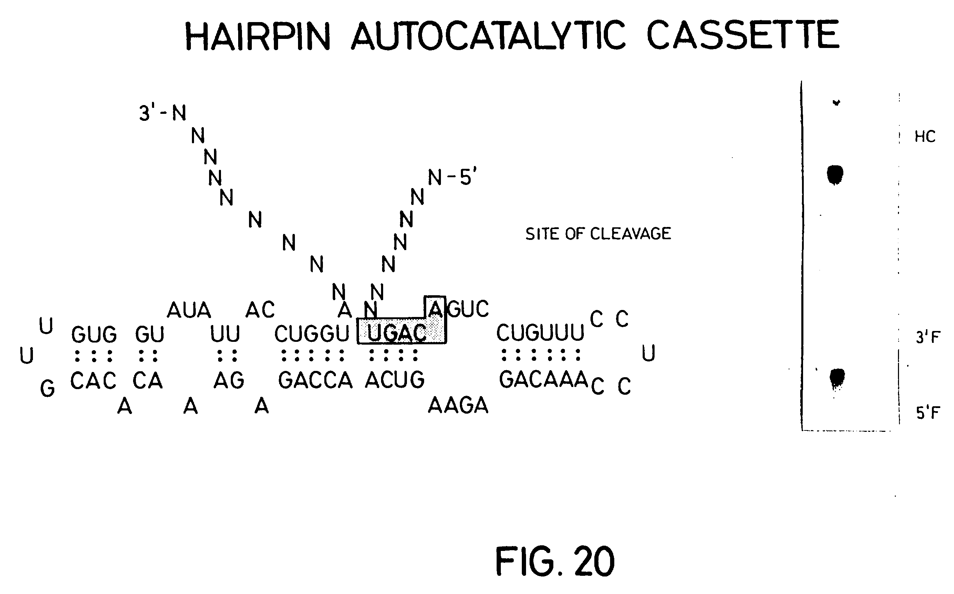

Figure 20. The RNA sequence of a synthetic "hairpin" autocatalytic cassette is shown. The sequence shown in Figure 20 is the same as that of the catalyst shown in Figure 1, but with additional 5'bases added to form a loop at the 5' end of the catalyst and to provide a substrate target sequence (i.e., a cleavage sequence and upstream and downstream flanking bases) which can bind to the substrate binding portion of the catalyst sequence. Such an RNA was prepared. When transcription was performed, the cassette autocatalytically cleaved at the expected site to give the appropriate 3'F and 5'F products.

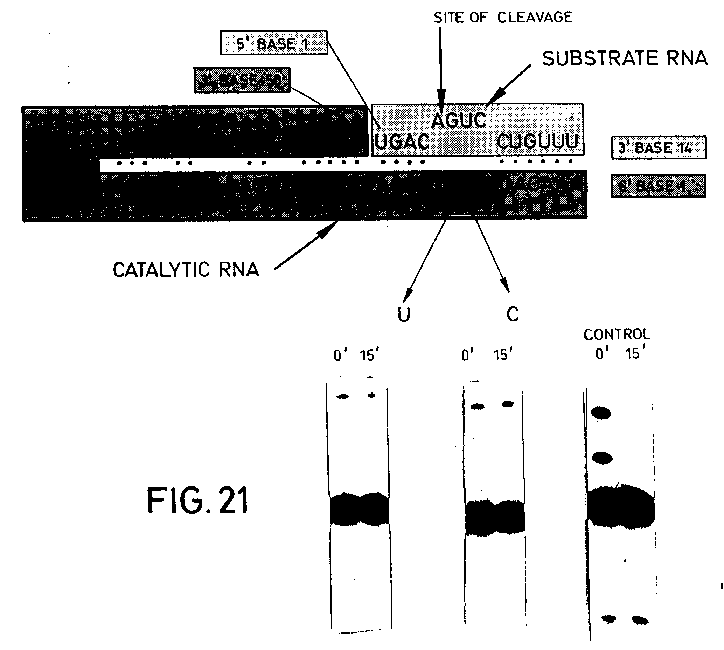

Figure 21. Shown are two base changes that were made in the native (-)sTRSV catalytic sequence shown in Figure 1. The two bases changes were an "A" to "U" mutation at position 217 and a "G" to "C" mutation at position 216. Figure 21 also shows the separation patterns on acrylamide gels of the reaction products obtained by reacting one of these catalysts or R51 (control) with substrate S17. Both base changes produced catalysts that were inactive when the catalysts were reacted with substrate S17 under standard conditions for 15 minutes.

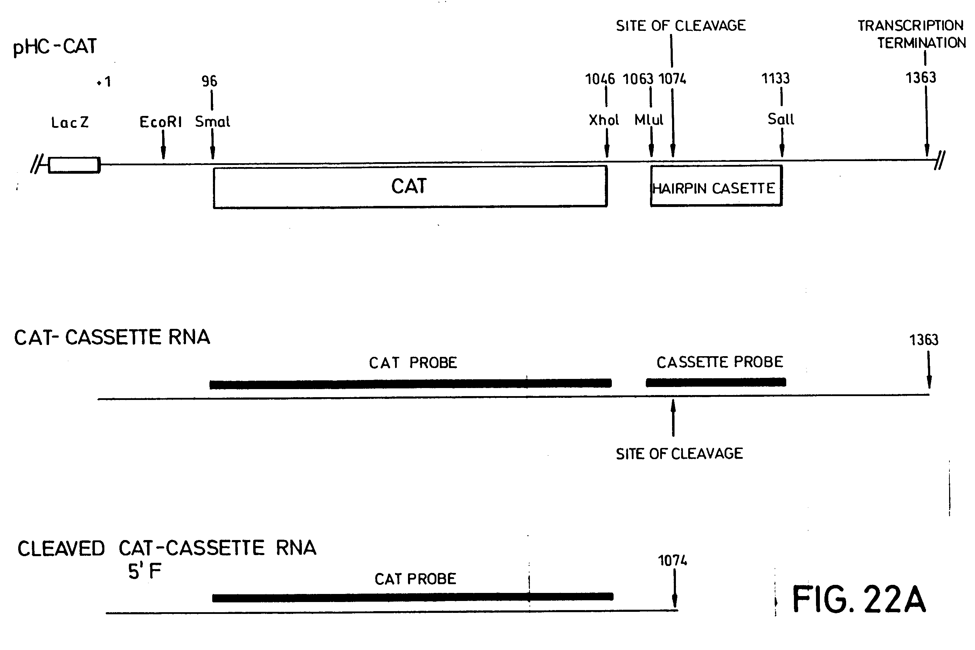

Figure 22A shows a partial map of plasmid pHC-CAT containing the CAT gene linked to the "hairpin" autocatalytic cassette of the invention. Also shown is the expected RNA transcript of the illustrated region and the expected 5' fragment of the autocatalytic cleavage.

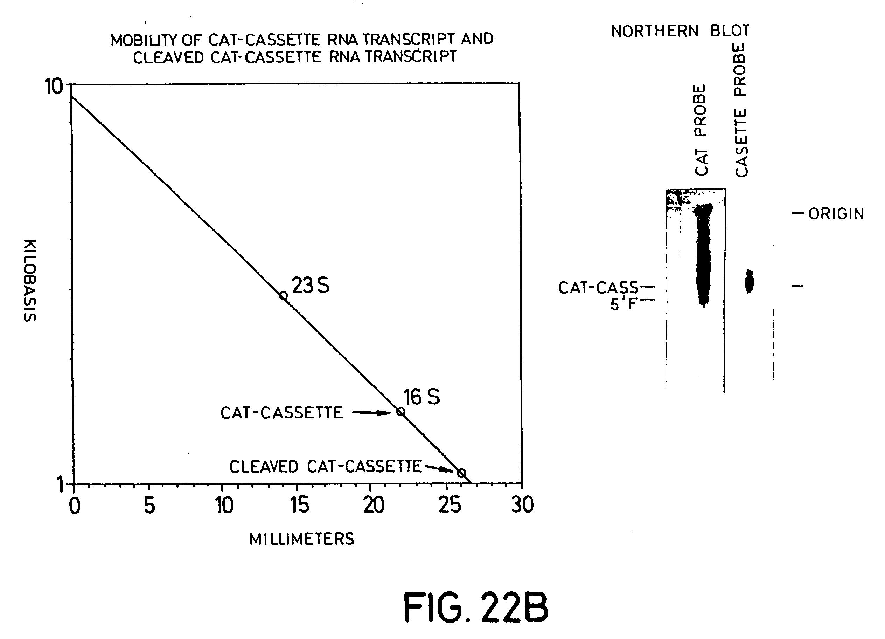

Figure 22B shows the result of Northern blot analysis of RNA produced by host cells transformed with pHC-CAT.

### DESCRIPTION OF THE PRESENTLY PREFERRED EMBODIMENTS

An RNA catalyst has been identified comprising an RNA sequence which can be engineered to cut, with great precision, target RNAs having a cleavage sequence. In particular, the invention comprises certain synthetic RNA catalysts capable of cleaving an RNA substrate which contains the sequence 5'-F₁-CS-F₂-3'. "Synthetic RNA catalyst," as used herein, means a catalyst which is not a naturally-occuring RNA catalyst, although "synthetic catalysts" may be truncated or altered versions of naturally-occuring catalysts. "Synthetic catalysts" include catalysts synthesized in vitro and catalysts synthesized in vivo. In particular, "synthetic catalysts" include catalysts produced by hosts transformed by a vector comprising a sequence coding for the catalyst.

RNA of any length and type may be used as the substrate as long as it contains the 5'-F₁-CS-F₂-3' target sequence. In this formula, CS is the cleavage sequence (i.e., a sequence of bases containing the site at which the catalyst cleaves the substrate). CS is a short sequence of bases which does not base pair with the RNA catalyst, and CS preferably has the sequence 5'-NGUC-3', wherein N is any base, and the substrate is cleaved by the catalyst between N and G to produce a fragment having an OH at the 5' end and a fragment having a 2',3' cyclic phosphate at the 3' end.

CS is flanked by two short base sequences F₁ and F₂ which do base pair with the RNA catalyst. F₁ is preferably at least 3 bases in length, most preferably 4 bases in length. F₂ is also preferably at least 3 bases in length, most preferably 6 to 12 bases in length.

Catalysts according to the invention comprise a substrate binding portion and a "hairpin" portion. The substrate binding portion of the catalyst has the sequence:
3'F₄-L₁-F₃-5'
wherein,
F₃ is a sequence of bases selected so that F₃ is substantially base paired with F₂ when the catalyst is bound to the substrate;
F₄ is a sequence of bases selected so that F₄ is substantially base paired with F₁ when the catalyst is bound to the substrate;
the sequences of F₃ and F₄ being selected so that each contains an adequate number of bases to achieve sufficient binding of the RNA substrate to the RNA catalyst so that cleavage of the substrate can take place; and
L₁ is a sequence of bases selected so that L₁ does not base pair with CS when the catalyst is bound to the substrate.

As used herein, "substantially base paired" means that greater than 65% of the bases of the two RNA sequences in question are base paired, and preferably greater than 75% of the bases are base paired. "Substantially unpaired" means that greater than 65% of the bases of the two sequences in question are not base paired, and preferably greater than 75% of the bases are not paired.

F₃ is preferably at least 3 bases in length, most preferably from 6 to 12 bases in length. F₄ is preferably from 3 to 5 bases in length, most preferably 4 bases in length. L₁ is a short sequence of bases which preferably has the sequence 5'-AGAA-3' when CS has the sequence 5'-NGUC-3'.

The "hairpin" portion is a portion of the catalyst which folds into a hairpin-like configuration when the substrate-catalyst complex is modeled in two dimensions for minimum energy folding. This is shown in Figures 1 and 2 for (-)sTRSV RNA. The "hairpin" portion is not an absolute hairpin in the sense that not all bases of the "hairpin" portion arc base-paired. Indeed, it is preferable, perhaps necessary, for the "hairpin" portion to have at least one substantially unpaired region so that the catalyst can assume a tertiary structure that allows for better, or optimal, catalytic activity.

The "hairpin" portion of the catalyst preferably has the sequence: 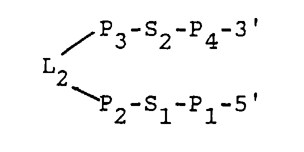 wherein,
P₁ and P₄ each is a sequence of bases, the sequences of P₁ and P₄ being selected so that P₁ and P₄ are substantially base paired;
P₁ is covalently attached to F₄;
S₁ and S₂ each is a sequence of bases, the sequences of S₁ and S₂ being selected so that S₁ and S₂ are substantially unpaired;
P₂ and P₃ each is a sequence of bases, the sequences of P₂ and P₃ being selected so that P₂ and P₃ are substantially base paired; and
L₂ is a sequence of unpaired bases.

"Substantially base paired" and "substantially unpaired" have the same meanings as discussed above.

P₁ and P₄ each is preferably from 3 to 6 bases in length, and most preferably P₁ has the sequence 5'-ACCAG-3' and P₄ has the sequence 5'-CUGGUA-3'.

S₁ and S₂ each preferably is from 4 to 7 bases in length, and most preferably S₁ has the sequence 5'-AGAA-3' and S₂ has the sequence 5'-AUAUUAC-3'.

P₂ and P₃ each is preferably from 3 to 6 bases in length, and most preferably P₂ has the sequence 5'-ACACAC-3' and P₃ has the sequence 5'-GUGGU-3'.

Finally, L₂ is preferably at least 3 bases in length and most preferably has the sequence 5'-GUU-3'. Further, 5'-S₁-P₂-L₂ preferably has the sequence 5'-AGAAACACACGUU-3'.

The specific preferred sequences set forth above for P₁, P₂, S₁, etc., are from the catalytic sequence of (-)sTRSV RNA.

The most preferred catalyst contains the sequence:
5'-F₃-L₁-F₄-ACCAGAGAAACACACGUUGUGGUAUAUUACCUGGUA-3',
and active variants thereof, wherein F₃, F₄ and L₁ are as defined above. As used herein "active variants" means catalysts which, although having substitutions, deletions and/or additions of bases as compared to the original sequence, are still capable of cleaving an RNA substrate.

Another preferred catalyst according to the invention is an autocatalytic catalyst containing the sequence: 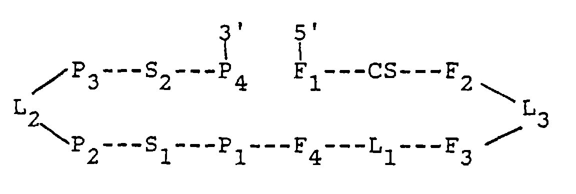 wherein, F₁, F₂, F₃, F₄, L₁, L₂, S₁, S₂, P₁, P₂, P₃ and P₄ are as defined above. Thus, the molecule contains a substrate portion (5'-F₁-CS-F₂-3') and a catalytic portion (5'-F₃-L₁-F₄-P₁-S₁-P₂-L₂-P₃-S₂-P₄-3') covalently linked together by L₃ so as to produce a synthetic autocatalytic RNA catalyst. L₃ is a sequence of unpaired bases, and L₃ preferably has the sequence 3'-CCUCC-5'.

After being transcribed, this catalyst will spontaneously undergo an intramolecular autocatalytic cleavage at CS. The effect of this intramolecular cleavage is to autocatalytically terminate any RNA transcript in which the sequence is inserted.

The invention further provides an engineered DNA molecule and a vector comprising a DNA sequence coding for an RNA catalyst of the invention. Also provided are host cells which have been transformed with the vectors and which are capable of expressing the RNA substrate. Finally, the invention provides a method of cleaving an RNA substrate which contains the sequence 5'-F₁-CS-F₂-3', the method comprising contacting the substrate with a synthetic RNA catalyst according to the invention.

The invention is further described below with particular reference to the catalytic properties of (-)sTRSV and the structure of its catalytic complex, but the invention is not limited thereto. In addition to the particular catalytic sequences shown and described below, other RNA molecules having catalytic activity to cleave an RNA substrate can be readily found by applying the principles set forth in this specification.

For example, RNA sequences having the required structural features for cleaving an RNA substrate can be identified by applying the Wisconsin RNA Folding Program discussed above to (1) known sequences of molecules having catalytic or autocatalytic activity (especially molecules in which the actual location of the catalytic site is unknown), (2) randomly generated sequences having the proper pairing regions and lengths, and (3) randomly modified known catalytic sequences, while looking for known features of a catalytic molecule, such as the "hairpin" configuration of the catalytic complex when modeled in two dimensions. As a specific example, information regarding known autocatalytic cleavage sites can be used to find substrate binding sequences having the properties described above, such as having a substrate binding sequence adjacent a "hairpin" portion. Secondary features, such as the two substantially paired regions in the "hairpin" portion, with an intermediate substantially unpaired region and an appropriate base loop can then be looked for, either by manual examination or with automated computer programs. In order of decreasing preference, the following features are considered important in selecting a catalytic sequence: (1) regions that can base pair (form helices) with the regions of the substrate RNA molecule flanking the cleavage sequence; (2) an unpaired (loop) region opposite the cleavage sequence of the substrate RNA; (3) two substantially base paired regions in a "hairpin" structure near the cleavage sequence; (4) a substantially unpaired region between these two substantially paired regions in the "hairpin" structure; and (5) a loop connecting the two strands of a substantially base paired region to complete the "hairpin" structure. Standard techniques of in vitro RNA synthesis can then be used to prepare actual molecules having the sequence that gives the predicted two-dimensional computer-generated structure for verification of activity and routine testing of variation to determine optimum sequence.

The catalysts of the present invention were developed using a "hairpin" model or motif of RNA catalysis. According to this model, the catalytic complex, when modeled in two dimensions for minimum energy folding, assumes a "hairpin" configuration. This is shown in Figure 1 for (-)sTRSV RNA. The catalytic complex is a complex of the minimum, or substantially the minimum, sequence of the catalyst necessary for activity and the minimum, or substantially the minimum, target sequence of the substrate. The "hairpin" configuration is not an absolute hairpin in the sense that not all the bases that make up this "hairpin" configuration are base-paired. Indeed, there are preferably regions of unpaired bases and of substantially unpaired bases as discussed in detail elsewhere in the present application.

The "hairpin" model has proved very useful in designing new catalysts, but it is still only a computer model of the likely secondary structure of catalytic complexes involving catalysts according to the present invention. Also, it is ultimately the tertiary structure of RNA catalysts that determines their activity. For these reasons, all catalysts having the properties described herein are considered to come within the scope of the present invention, even if they do not form a "hairpin" configuration when complexed with the substrate and even if they do not contain a "hairpin" portion. For instance, it may be possible to engineer a catalyst having the properties described herein which does not have a loop L₂. Such a catalyst would be considered to be fully equivalent to the catalysts described and claimed herein.

As described in Example 1, a catalytic complex was identified within the (-) strand of tobacco ringspot virus satellite RNA (sTRSV) when the molecule was folded using computer models to determine the minimum energy folding in two-dimensional space. The (-) strand is a 359 base long RNA of defined sequence and is known to have autocatalytic properties (Gerlach, W. L., Buzayan, J. W., Schneider, I. R. and Bruening, G. B. (1986) Virology, 151: 172-185; Buzayan, J. M., Gerlach, W. and Bruening, G. (1986) Nature, 323: 349-352). The (-) strand cleaves at a defined site (ApG) into a cleavage product having an OH at the 5' end and a 2',3' cyclic phosphate at the 3' end. Up until the present time, however, little work had been done with the (-) strand to find the catalytic complex and to determine the minimum cleavage sequences because it does not fit the consensus "hammerhead" model.

In view of the above and the fact that the catalytic center would contain both the catalytic RNA sequence and the substrate (target) RNA sequence and by studying the results of Example 1, a 50 nucleotide sequence between bases 175 and 224 was picked and a 14 nucleotide sequence between bases 40 and 53 was picked. Using methodologies found in published procedures, a catalytic RNA having a satellite RNA base sequence identical to the base sequence in naturally occurring (-)sTRSV between bases 175 and 224 was transcribed from chemically synthesized DNA templates using T7 RNA polymerase as described in Example 2. An RNA substrate having a satellite RNA base sequence identical to the base sequence in naturally Occurring (-)sTRSV RNA between bases 40 and 53 was also prepared in the same manner. When the newly synthesized RNAs were mixed together under appropriate conditions as described in Example 3, the catalytic RNA cleaved the substrate RNA. As described in Example 4, the first RNA catalyst fitting the "hairpin" motif was discovered when the complex of the 50-base catalytic RNA and the 14-base substrate RNA were modeled in two-dimensional space using computer modeling.

The reaction of catalysts fitting the "hairpin" motif with an appropriate substrate proved to be an excellent catalytic reaction under physiological conditions. The reaction of the catalyst and substrate containing the sequences of (-)sTRSV shown in Figure 1 gave a Km of 0.03uM (see Example 5), which is 20 times smaller than that of the Km for a catalyst fitting the "hammerhead" model (Uhlenbeck, O. C. (1987) Nature 328: 596-600) and accounts for its ability to remove target RNA molecules to much lower levels (20 times lower) than that of catalysts fitting the "hammerhead" model. In addition, the kcat for the reaction is 2.1/min at 37°C, which is at least 6 times greater than that of a catalyst having the "hammerhead" configuration at the same temperature (see Example 5). These reaction parameters for a catalyst that fits the "hairpin" model can be further improved by increasing the amount of base pairing between the substrate and catalyst (see Example 21).

Catalytic cleavage of the substrate RNA occurs over a broad pH range, preferably 5.5 to 8.0, and in the presence of divalent ions such as Mg++, e.g. from MgCl₂. As would be expected for a base catalyzed reaction, the rate of reaction increased with increasing pH. The reaction rate also increased with increasing concentration of divalent cations as shown in Example 8.

The reaction takes place at physiological temperatures, preferably 16°C to 45°C, with a temperature optimum at 37°C as described in Example 6. Temperatures above about 45°C inactivate the reaction. Also, the temperature optimum of the reaction is affected by the degree of base pairing between the substrate and catalyst (see Example 18). In particular, the length of the region of the catalyst that base pairs with the 3' region of the substrate flanking the cleavage sequence can be varied so that an engineered catalyst reacting at a desired temperature can be obtained (see Example 18).

The 50 base catalytic RNA configured in the "hairpin" model in Fig. 1 is the minimal sequence, or substantially the minimal sequence, of (-)sTRSV RNA necessary to achieve catalysis. When the 3' terminal A (base 175) or AU (bases 175 and 176) were removed, catalytic activity was deceased by 75% as shown in Example 10. The 5' end of the molecule cannot be substantially changed either without affecting catalytic activity because it is needed to provide base pairing with the substrate. It can be shortened by at most about 3 bases (see Example 9). Experiments which removed bases 195-203 in the center of the catalytic RNA and ligated base 194 to base 204 produced an inactive catalytic RNA as described in Example 11. This shows that all or part of the region between bases 195-203 is essential for catalytic activity. An additional mutagenesis experiment to test the base requirement of the three A bases at positions 203, 202 and 201 was done. When these bases were changed to CGU, respectively, as described in Example 12, the resulting catalytic RNA was inactive.

Base changes can be made in the two base paired regions of the "hairpin" portion of the catalytic RNA, as long as substantial base pairing is maintained. This is shown in Example 22 where base changes that destroyed base pairing in these two regions resulted in inactive catalysts. When a second base change was made which restored base pairing, the catalytic activity was also restored (see Figure 19).

Base changes can also be made in the two regions of the catalytic RNA that base pair with the substrate, as long as substantial base pairing with the substrate in the regions flanking the cleavage sequence is maintained and base pairing with the cleavage sequence is avoided as shown in Examples 9 and 16-21. It is this feature that gives the RNA catalyst flexibility to be engineered to cut a specific target RNA substrate having a cleavage sequence such as NGUC. This is illustrated in Example 16 where the catalytic RNA was engineered by changing base 214 from & G to a C resulting in a catalytic RNA which failed to react with the substrate RNA developed from natural (-)sTRSV RNA. Activity was restored, however, when the substrate RNA was changed so that it could base pair with the subject engineered catalytic RNA. Also see Examples 9 and 17-21.

The (-)sTRSV catalytic RNA sequence has an 5'-AGAA-3' sequence opposite the AGUC cleavage sequence of the substrate. As shown in Example 24, at least part of this AGAA sequence seems to be invariant. In particular, the base at position 217 in the AGAA loop was changed from a G to a C, and the base at position 216 was changed from A to U (see Figure 21). As reported in Example 24, these changes destroyed the activity of the catalyst.

The target RNA substrate of the "hairpin" catalytic complex shown in Fig. 1 has an AGUC cleavage loop which does not base pair to the catalytic RNA in two-dimensional space. As shown in Examples 13-15, "A" in the the AGUC cleavage sequence can be changed to any other base without effecting the ability of the RNA catalyst to cleave the substrate. Example 25 shows that the GUC sequence is conserved. Thus, the only sequence requirement for the cleavage sequence of the substrate RNA is GUC.

The cleavage sequence has four flanking bases at its 5' end and six at its 3' end which base pair with the catalytic RNA. As described above, the bases in the flanking regions can be changed without affecting the ability of the catalytic RNA to cleave the substrate as long as sufficient base pairing with the catalyst is maintained in the flanking regions. This would be expected to work on longer RNA substrate sequences of any length as long as these criteria are met. Indeed, lengthening the 3' region of the substrate that base pairs with the catalyst has been found to provide a more efficient catalytic reaction. See Examples 18 and 21. However, a smaller 10 base substrate having three flanking bases at its 5' end and three flanking bases at its 3' end did not work as well as the 14-base substrate, as described in Example 9.

Using the "hairpin" model as a guide, RNA catalysts can be engineered that base pair with an RNA substrate and mediate a cleavage in the cleavage sequence. In particular, catalytic RNA can be engineered that will cleave any RNA substrate having a cleavage sequence, such as NGUC, and flanking regions with which the catalyst base pairs, so that the catalytic RNA and RNA substrate form a catalytic complex in a "hairpin" motif. To do this, the bases flanking the cleavage sequence must be identified and the catalytic RNA engineered so that it does not pair in two-dimensional space with the cleavage sequence but does pair with adequate numbers of flanking bases upstream and downstream of the cleavage sequence.

As shown in Examples 17-20, catalytic RNAs according to the invention can cleave specific viral and messenger RNA sequences. In Example 15, tobacco mosaic virus (TMV) RNA containing the 5' coding region of the replicase gene was targeted for specific cleavage by an appropriately engineered catalytic RNA. The target sequence contained changes in 8 of the 14 bases of the substrate RNA having a base sequence found within the catalytic complex of (-)sTRSV RNA and was cleaved by the engineered RNA catalyst under conditions near physiological. Catalytic RNAs were also designed and synthesized using the "hairpin" model as a guide which could cleave sequences from messenger RNA coding for chloramphenicol acetyl transferase (Example 18) and from HIV-1 viral RNA coding for the gag and the tat proteins (Examples 19 and 20).

These examples demonstrate that the system can be used to specifically cleave an RNA sequence for which an appropriately engineered catalytic RNA base pairs at the designated flanking regions. Suitable target RNA substrates include viral, messenger, transfer, ribosomal, nuclear, organellar, other cellular RNA, or any other natural RNA having a cleavage sequence, as well as RNAs which have been engineered to contain an appropriate cleavage sequence.

Catalysts that fit the "hairpin" catalytic RNA model are expected to be useful in vivo in prokaryotes or eukaryotes of plant or animal origin for controlling viral infections or for regulating the expression of specific genes. In this case, a cleavage sequence such as NGUC in the virus or complementary to NGUC in the gene would need to be identified along with the flanking sequences immediately upstream and down-stream of the cleavage sequence. Normally three to four bases on the 5' side of the cleavage sequence and enough bases in the order of 6 to 12 on the 3' side to provide adequate binding of the catalytic RNA and to provide reasonable certainty that the target RNA sequence is unique in the organism are all that is required.

A catalytic RNA is then engineered which does not base pair with the cleavage sequence and which does base pair to the flanking regions on the 5' and 3' side of the cleavage sequence. A DNA template corresponding to this catalytic RNA is then synthesized using procedures that are well-known in the art. Such procedures include the phosphoramidite method.

The invention also includes an engineered DNA molecule and a vector comprising a sequence coding for the desired RNA catalyst. The vector will have the DNA sequence coding for the desired catalytic RNA operatively linked to appropriate expression control sequences. Methods of effecting this operative linking, either before or after the DNA coding for the catalyst is inserted into the vector, are well known. Expression control sequences include promoters, activators, enhancers, operators, stop signals, cap signals, polyadenylation signals, and other signals involved with the control of transcription.

The vector must contain a promoter and a transcription termination signal, both operatively linked to the synthetic DNA sequence, i.e., the promoter is upstream of the synthetic DNA sequence and the termination signal is downstream from it. The promoter may be any DNA sequence that shows transcriptional activity in the host cell and may be derived from genes encoding homologous or heterologous proteins and either extracellular or intracellular proteins, such as amylase, glycoamylases, proteases, lipases, cellulases, and glycolytic enzymes. Also, a promoter recognized by T7 RNA polymerase may be used if the host is also engineered to contain the gene coding for T7 RNA polymerase.

The promoter may contain upstream or down-stream activator and enhancer sequences. An operator sequence may also be included downstream of the promoter, if desired.

Expression control sequences suitable for use in the invention are well known. They include those of the E. coli lac system, the E. coli trp system, the TAC system and the TRC system; the major operator and promoter regions of bacteriophage lambda; the control region of filamentous single-stranded DNA phages; the expression control sequences of other bacteria; promoters derived from genes coding for Saccharomyces cerevisiae TPI, ADH, PGK and alpha-factor; promoters derived from genes coding for the Aspergillus oryzae TAKA amylase and A. niger glycoamylase, neutral alpha-amylase and acid stable alpha-amylase; promoters derived from genes coding for Rhizomucor miehei aspartic proteinase and lipase; mouse mammary tumor promoter; SV40 promoter; the actin promoter; and other sequences known to control the expression of genes of prokaryotic cells, eukaryotic cells, their viruses, or combinations thereof.

The vector must also contain one or more replication systems which allow it to replicate in the host cells. In particular, when the host is a yeast, the vector should contain the yeast 2u replication genes REP1-3 and origin of replication.

The vector should further include one or more restriction enzyme sites for inserting the DNA template sequences into the vector, and preferably contains a DNA sequence coding for a selectable or identifiable phenotypic trait which is manifested when the vector is present in the host cell ("a selection marker").

Suitable vectors for use in the invention are well known. They include retroviral vectors, vaccinia vectors, pUC (such as pUC8 and pUC4K), pBR (such as pBR322 and pBR328), pUR (such as pUR288), phage lambda, YEp (such as YEp24) plasmids, and derivatives of these vectors.

The resulting vector having the synthetic DNA sequence that codes for the RNA catalyst is used to transform an appropriate host. This transformation may be performed using methods well known in the art.

Any of a large number of available and well-known host cells may be used in the practice of this invention. The selection of a particular host is dependent upon a number of factors recognized by the art. These include, for example, compatibility with the chosen expression vector, toxicity to it of the catalytic RNA encoded for by the synthetic DNA sequence, rate of transformation, expression characteristics, bio-safety and costs. A balance of these factors must be struck with the understanding that not all hosts may be equally effective for the expression of a particular catalytic RNA.

Within these general guidelines, useful hosts include bacteria (such as E. coli sp.), yeast (such as Saccharomyces sp.) and other fungi, insects, plants, animals (including human), or other hosts known in the art.

As an example of the general genetic engineering techniques that are possible, mammalian vectors can be used to deliver DNA coding for the catalytic RNAs of the invention to animal cells. These vectors should have a suitable DNA replication signal, such as from SV40, a promoter which may or may not be inducible, such as the mouse mammary tumor promoter (which is induced by dexamethasone) or the noninducible SV40 promoter. A multiple cloning site is located after the promoter, and the DNA coding for the engineered catalytic RNA is inserted into this multiple cloning site using standard techniques. If necessary a suitable terminator is inserted. The resulting vector is then put into cells using standard techniques such as direct injection into the nucleus, electroporation, or other well-known transformation techniques. Once the vector is in the cell, the catalytic RNA is expressed directly when noninducible promoters are used, or after addition of the inducer when inducible promoters are used.

Similarly, plant vectors, such as the Ti plasmid or micro-Ti plasmids, can be used to deliver DNA coding for a desired catalytic RNA to plant cells. The Ti plasmids and micro-Ti plasmids may be used as such to transform plant protoplasts using known techniques or may be inserted into Agrobacterium tumefaciens which is then used to transform plant tissue. Once the plasmid is in the cell, the catalytic RNA will be expressed.

Once transformed, a host cell can express (transcribe) the RNA catalyst. When the DNA coding for the catalyst is transcribed, it produces catalytic RNA which will attack and cleave the target RNA sequence or sequences for which it has been designed, inactivating the RNA. If the RNA is necessary for the life cycle of a virus, the virus will be eliminated and if the RNA is the product of a specific gene, the expression of that gene will thus be regulated. The catalytic RNA can be designed to work in prokaryotes and within the nucleus (without polyA tail) or in the cytoplasm of a eukaryotic cell (with polyadenylation signals in place) for plants and animals.

Another potential method of using the catalytic RNAs of the invention is to prepare stable synthetic derivatives of RNA catalysts designed to bind and cleave a specific target RNA and to deliver the modified catalysts directly to the cell or organism of choice. For example, standard methods are available for making phoshporothioate derivatives of DNA which have been shown to be very stable in vivo and to be able to bind to a specific DNA or RNA target in vivo (antisense method). A modification of these procedures can be used to prepare a catalytically active derivative of RNA catalysts prepared according to the invention. This would entail determining which ribonuclerotide regions can be altered and then altering them with deoxy, phophorothio, or other modifications which confer stability but do not destroy catalytic activity. This chemically modified catalytic RNA (which may or may not have any remaining RNA bonds) can then be injected or otherwise delivered to an organism to control viruses or gene expression. For instance, one of the catalytic RNAs whose preparation is described in Examples 19-20 having specificity for sequences found within the RNA of the HIV-1 virus that causes AIDS could be chemically modified as described, encapsulated in a liposome coated with monoclonal antibody directed to the CD4 receptors found on cells susceptible to HIV-1, and injected into a host animal.

The "hairpin" catalytic RNA model of the present invention may also be of possible interest to molecular biologists in exploring the genetic blueprints of plants and animals. This would be done by sending randomly constructed DNA reverse transcripts of catalytic RNA into the DNA of the organism and waiting to see which gene or genes were inactivated. Other techniques could be applied to determine where those genes resided on the organism's chromosomes, thereby greatly accelerating gene mapping.

Finally, a synthetic autocatalytic RNA catalyst has been developed. The synthesis of one such catalyst based on the (-)sTRSV RNA catalytic and substrate sequences is described in Example 23. The use of such autocatalytic catalysts allows for the efficient autocatalytic termination of any RNA transcript in which it is inserted.

### EXAMPLES

The following examples illustrate the invention.

### Example 1

The (-) sense sequence of satellite RNA from the budblight strain of tobacco ringspot virus as shown in Fig. 2 was folded using the Wisconsin RNA folding program to identify the location of a possible catalytic complex accounting for its ability to self cleave (University of Wisconsin Genetics Computer Group, Program FOLD 5/6/86) (Zucker, M. and Stiegler, P. (1981) Nucleic Acids Res., 9: 133-148; Devereux, J., Haeberli, P. and Smithies, O. (1984) Nucleic Acids Res., 12: 387-395). Base numbers correspond to (+)sTRSV RNA (Buzayan, J. M., Gerlach, W. L., Bruening, G. B., Keese, P. and Gould, A. R. (1986) Virology, 151: 186-199). With this numbering scheme the 5'-3' direction of the molecule is with decreasing base number.

The minimum catalytic complex, or active site of the molecule, is identified in Figure 2. The folding identified regions of expected base pairing which are in classical double-helical or stem regions. The folding also identified expected non-base pairing loops at or near the site of cleavage. This model does not preclude higher order interactions occurring between non-adjacent portions of the catalytic center.

### Example 2

A 50 nucleotide sequence between bases 175 and 224 was picked and a 14 nucleotide sequence between bases 40 and 53 was picked from the catalytic complex identified in Example 1. A catalytic RNA (R51) with the 50 base sequence shown in Fig. 1 plus one additional vector base (G at the 5' end) and a substrate RNA (S17) with the 14 base sequence shown in Fig. 1 plus three vector bases (GCG at the 5' end) were transcribed using T7 RNA polymerase from synthetic DNA templates double stranded at the promoter site (Milligan, J. F., Groebe, D.R., Witherell, G. W. and Uhlenbeck, O. C. (1987) Nuc. Acids Res., 15: 8783-8798). The synthetic DNA templates were made using phosphoramidite chemistry on an Applied Biosystems 381A DNA synthesizer. The template DNAs were: catalytic RNA R51: 3'ATTATGCTGAGTGATATCTTTGTCTCTTCAGTTGGTCTCTTTGTGTGCAACACCATATAATGGACCAT 5' and substrate RNA 517: 3'ATTATGCTGAGTGATATCGCACTGTCAGGACAAA 5'.

Before transcription, a 18mer or 16mer DNA complement to the promoter for T7 RNA polymerase on the noncoding strand was hybridized by heating an equimolar amount of template DNA with promoter complement to 65°C for 3 min. then placing in ice. A typical transcription reaction used 8ng/ul DNA template, 0.5mM each NTP, 2mM spermidine, 40mM Tris pH 7.5, 4% polyethylene glycol 6,000, 6mM MgC1₂, 4mM NaC1, 10mM dithiothreitol, 0.01% Triton X-100, 2.4 units/ul RNasin, 1.8uCi/ul P³² CTP and 3 units/ul T7 RNA polymerase (US Biochemical) and was run at 37°C for 90 min.

All in vitro transcribed RNAs were isolated on 7M urea, 15-20% acrylamide gels, bands cut out and isolated. All RNAs were sequenced using standard methods (Donis-Keller, H., Maxam, A. M. and Gilbert, W. (1980) Nucleic Acids Res., 4: 2527-2538); a method which also gave the 5' terminal base. Terminal bases at the 3' end were determined by ligation of the RNA to 5' P³² pCp using T4 RNA ligase (BRL methods manual), nuclease T2 digestion, and separation of labelled bases by PEI thin layer chromatography in 0.3M LiC1 with appropriate standards. All RNA sequences corresponded to that expected from the DNA template.

### Example 3

The catalytic RNA R51 was added to the substrate RNA S17 at a ratio of 1:30 and the time course of substrate RNA cleavage was studied. The reaction was carried out at 37°C in 12mM MgC1₂, 40mM Tris pH 7.5 and 2mM spermidine over a time period of 150 min and is summarized as follows:

5'gcgUGACAGUCCUGUUU 3'    R51    gcgUGUCA + GUCCUGUUU
  (S17)                          (5'F)       (3'F)

The reaction products were separated on polyacrylamide/urea gels by electrophoresis and bands cut out and counted in a liquid scintillation counter. The results are shown in Fig. 3. The time periods analyzed were: lane 1, 30 Sec; lane 2, 5 min; lane 3, 15 min; lane 4, 30 min; lane 5, 60 min; lane 6, 90 min and lane 7, 150 min. Beginning concentrations were as follows: R51=0.0032uM and S17=0.09uM.

As shown in Fig. 3, the cleavage proceeds to virtual completion during the course of the reaction with only 2% of the substrate remaining after 150 minutes. This shows that, since there was originally 30 times as much substrate RNA as catalytic RNA, the RNA catalyst R51 of necessity interacts with multiple substrate molecules during the course of the reaction. In addition, the amount of catalyst remained the same and was unaltered, indicating that R51 is truly a catalytic entity.

### Example 4

After the RNA catalyst had been shown to be effective in cleaving the RNA substrate as described in Example 3, minimum energy folding of the 50 base sequence shown in Fig. 1, complexed with the 14 base sequence was done using the computer methods described in Example 1. The folded complex forms a "hairpin" model or motif as shown in Fig. 1 with the substrate RNA sequence and the catalytic RNA sequence identified. The arrow is at the site of cleavage.

### Example 5

Various concentrations of substrate S17 were used at constant concentration of catalyst R51 and initial velocities of each reaction determined. The reaction was carried out at 37°C in 12mM MgC1₂, 40mM Tris pH 7.5 and 2mM spermidine. Concentrations were as follows: R51=0.0004uM and S17=0.125uM (lane 1), 0.0624uM (lane 2), 0.0417uM (lane 3), 0.031uM (lane 4), 0.021uM (lane 5), 0.0156uM (lane 6), 0.0078uM (lane 7) and 0.0039uM (lane 8). Each reaction was analyzed on polyacrylamide gels as described in Example 3 with the results shown in Fig. 4. An Eadie Hofstee plot of catalytic RNA R51 cleavage of substrate RNA S17 is shown in Fig. 4. The reaction proceeded according to the predictions of the Michaelis-Menten equation indicating that it was a truly enzymatic reaction in that as the concentration of the substrate goes down, the velocity of the reaction goes down. From the velocity of the reaction plotted as a function of substrate concentration, the Km calculated from the graph was 0.03uM and the kcat (turnover number) was 2.1/min. This Km is 20 times smaller than the "hammerhead" catalysts (see Uhlenbeck, O. C. (1987) Nature, 328: 596-600) indicating that lower concentrations of substrate can be removed. The kcat is 6 times larger than that of catalysts fitting the "hammerhead" model at 37^{o}C (see Uhlenbeck, O. C. (1987) Nature, 328: 596-600), meaning that the reaction is 6 times faster.

### Example 6

The temperature dependence of the rate of cleavage of substrate RNA S17 by catalytic RNA R51 was tested over a temperature range and the reaction products analyzed on polyacrylamide gels as described in Example 3 with the results shown in Fig. 5. The reaction was carried out in 12mM MgC1₂, 40mM Tris pH 7.5 and 2mM spermidine at 45^{o}C (lane 6), 41°C (lane 5), 37°C (lane 4), 33°C (lane 3), 27°C (lane 2) and 20°C (lane 1). The concentrations used were: R51=0.0016uM and S17=0.04uM. R51 was unlabeled. The velocities shown in the graph in Figure 5 were calculated by the use of time points of 8 and 16 minutes. The separation patterns on the gel shown in Figure 5 is for the 16-minute time point.

The reaction showed a temperature dependence similar to that which would be expected of a reaction involving base paired RNA molecules. The Arrhenius plot of the data shown in Fig. 5 gives a temperature optimum of 37°C for the reaction. Higher temperatures reduce the reaction rate with a very rapid rate reduction above 41°C consistent with a melting out of the catalytic RNA structure. At 50°C no reaction was detectable. The reaction rate at temperatures below 37°C showed a linear reciprocal temperature dependence consistent with a classical lowering of the energy of activation for the reaction. The slope of the line in the Arrhenius plot gave an energy of activation of 19 Kcal/mole which is close to that found for catalysts fitting the "hammerhead" cleavage mechanism (13.1 Kcal/mole) (Uhlenbeck, O. C. (1987) Nature, 328: 596-600).

### Example 7

The rate of cleavage of a constant concentration of substrate RNA S17 at varying concentrations of catalytic RNA R51 was tested and the reaction products analyzed on polyacrylamide gels as described in Example 3 with the results shown in Fig. 6. The reaction was carried out at 37°C in 12mM MgCl₂, 40mM Tris pH 7.5 and 2mM spermidine for 40 min (lane 1 and 2), 20 min (lane 3), 10 min (lane 4) and 5 min (lane 5). The concentration of substrate was 0.175uM. The results are plotted in Fig. 6 and show that at saturating concentrations of substrate the reaction rate is linear with increasing RNA catalyst concentrations as one would expect for a true catalytic reaction.

### Example 8

The effect of Mg⁺⁺ concentration and pH on the rate of cleavage of RNA substrate S17 by RNA catalyst R51 was determined as shown in the following table:

| MgC1₂(mM) | t_{1/2}(min) |
|---|---|
| 0 | no detectable product |
| 4 | 136 |
| 6 | 111 |
| 8 | 115 |
| 10 | 88 |
| 12 | 81 |
| 15 | 74 |
| 20 | 62 |

| pH | t_{1/2}(min) |
|---|---|
| 5.5 | 330 |
| 6.0 | 120 |
| 6.5 | 67 |
| 7.0 | 48 |
| 7.5 | 42 |
| 8.0 | 38 |

In the Mg⁺⁺ studies, the substrate S17 concentration was 0.14uM and RNA catalyst R51 concentration was 0.0015uM. The reactions were at 37°C in 40mM Tris pH 7.5. In the pH studies, the substrate S17 concentration was 0.062uM and RNA catalyst R51 concentration was 0.0014uM. The reactions were at 37°C in 40mM Tris for pH 7.0, 7.5, 8.0 and in 40mM Pipes for pH 5.5, 6.0 and 6.5.

The dependence of the reaction rate on Mg⁺⁺ and pH are virtually identical with those of catalysts fitting the "hammerhead" model. The reaction rate increases with increasing pH as one would expect for a base catalyzed reaction but the effect is masked by the catalytic activity of the RNA. Hence a 100 fold increase in [OH⁻] between pH 6.0 and 8.0 resulted in only a 3 fold increase in the reaction rate.

### Example 9

A 10 base substrate (S10) was prepared by the methodology of Example 2. When the substrate was mixed with catalytic RNA R51, the reaction is summarized as follows:

5'GACAGUCCUG 3'     R51     GACA     +     GUCCUG
(S10)                        (5'F)          (3'F)

The results of rate studies with substrate S10 comparable to those described with S17 in Example 5 showed a Km=0.06uM and a kcat=0.8/min. These results are shown in Fig. 7 and indicate that smaller substrates can be used, but not as efficiently.

### Example 10

The 3' terminal base of the catalyst shown in the "hairpin" model of the (-)sTRSV catalytic complex in Fig. 1 is at position 175. Two catalytic RNAs were prepared with exactly the same sequence as R51, except that one of them did not contain the 3' terminal "A" base (position 175) and the other one did not contain the 3' terminal "UA" sequence (positions 176 and 175). Synthesis of these catalytic RNAs, designated R50 and R49, respectively, was carried out as described in Example 2. R50 or R49 catalytic RNA was mixed with substrate RNA S17 under standard conditions of reaction and the products analyzed as described in Example 3. The results are given in Fig. 8 and show a 75% reduction in activity with either R50 or R49 as compared to the activity of catalytic RNA R51 having the 3' terminal "A" at position 175 and the 3' terminal "AU" at positions 175 and 176.

### Example 11

An RNA with the same sequence as catalytic RNA R51 was prepared, except that bases 195-203 were omitted such that base 194 was in effect ligated to base 204. This RNA molecule was prepared as described in Example 2 from an appropriate DNA template containing the complementary sequence. When this RNA was mixed with substrate RNA S17 as described in Example 3, no reaction occurred. These results show that major elements of the "hairpin" structure are required for RNA catalysis to occur and that removal of 9 bases (see Fig. 9) in the middle inactivates the catalytic RNA.

### Example 12

An RNA with the same sequence as R51, except that the bases AAA at positions 203, 202 and 201 were changed to CGU, respectively, was prepared as described in Example 2 using an appropriate DNA primer. When this potential RNA catalyst was mixed with substrate RNA S17 as described in Example 3, no reaction occurred. This shows that the integrity of one or all of these bases (see Fig. 10) is required for catalytic activity.

### Example 13

A substrate RNA with the base at position 49 in Fig. 1 changed from an "A" to a "G" was prepared as described in Example 2. When this substrate was reacted with the RNA catalyst R51, no difference in rate of reaction was seen between this substrate and the substrate containing the "A" at position 49 (see Fig. 11A). This shows that alterations can occur in the "A" base in the substrate RNA AGUC loop without affecting the ability of the catalytic RNA to cleave the substrate.

### Example 14

Another substrate RNA identical to S17 but having "A" replaced by "U" in the AGUC loop was prepared as described in Example 2 (designated "S17(A-->U)"). This substrate RNA, at a concentration of 0.12uM, was reacted with the catalytic RNA R51, at a concentration of 0.0065uM, under standard conditions as described in Example 3 for 60 minutes. The results are shown in Figure 11B where Lane 1 contains the products of the reaction of substrate RNA S17(A-->U) with R51 catalytic RNA. No difference in the rate of reaction was seen between S17(A-->U) substrate RNA and substrate S17 containing the "A" base at position 49.

### Example 15

Another substrate RNA identical to S17 but having "A" replaced by "C" in the AGUC loop was prepared as described in Example 2 (designated "S17(A-->C)"). This substrate RNA, at a concentration of 0.08uM, was reacted with the catalytic RNA R51, at a concentration of 0.0065uM, under standard conditions as described in Example 3 for 60 minutes. The results are shown in Figure 11C where Lane 1 contains the products of the reaction of S17(A-->C) substrate RNA with R51 catalytic RNA. Again, no difference was seen in the rate of reaction using S17(A-->C) as compared to S17 containing the "A" base at position 49. The combined results of Examples 13-15 show that the base at position 49 in the cleavage sequence of the substrate may be any base.

### Example 16

Base changes in the stem regions at the site of binding of the substrate RNA to the catalytic RNA in the "hairpin" configuration can be made as long as the base pairing is maintained. The "C" base at position 50 of the substrate was changed to a "G" using the methods in Example 2. When this substrate RNA was reacted with the catalytic RNA R51, no cleavage of this substrate occurred. However, when a new catalytic RNA, containing a "C" at position 214, rather than the "G" found in R51, was synthesized according to the methods in Example 2 and added to this substrate, full cleavage was seen. The effect of the base change from "C" to "G" in the substrate was to eliminate the base pairing at this position predicted by the "hairpin" model since now a "G" would be across from a "G". However, when a "G" to "C" base change was made in the catalytic RNA, the base pairing was restored, but in a reverse manner, and the integrity of the helices in the stem regions where the substrate RNA binds to the catalytic RNA was thus conserved restoring catalytic activity (see Fig. 12).

### Example 17

An RNA sequence found within the sequence of tobacco mosaic virus was synthesized using the methods described in Example 2. This synthesized target RNA had the sequence 5'gAAACAGUCCCCAAC 3'. A catalytic RNA was synthesized with the sequence 5'GUUGGGAGAAGUUUACCAGAGAAACACACGUUGUGGUAUAUUACCUGGUA3' selected so that base pairing between the substrate and the catalytic RNA is maintained in the "hairpin" configuration (see Fig. 13). When these two RNAs were mixed under standard catalytic conditions as described in Example 3, the target was cleaved demonstrating that a sequence found within a native RNA can be cleaved.

### Example 18

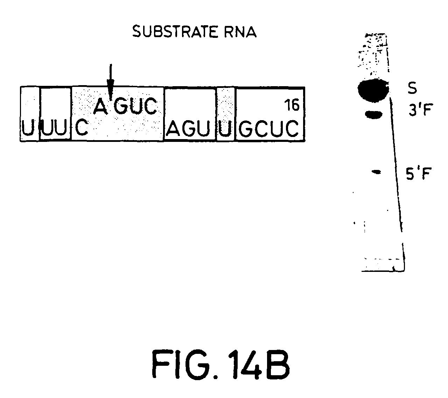

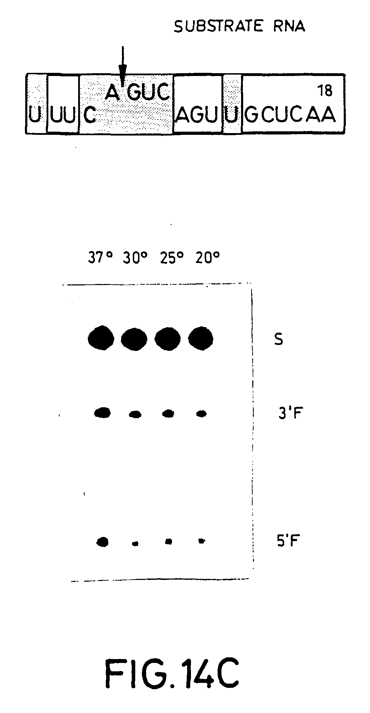

Three RNA sequences found within the sequence of the messenger RNA for the enzyme chloramphenicol acetyl transferase (CAT) were sythesized using the methods described in Example 2. The synthesized substrate RNAs had the sequences (A) gUUUCAGUCAGUUGC; (B) gUUUCAGUCAGUUGCUC; and (C) gggUUUCAGUCAGUUGCUCAA (see Fig. 14).

Note that the latter two sequences are extensions of the first sequence and that additional bases have been added to the 3' end in the region that the "hairpin" model predicts will base pair with the catalytic RNA to form Helix 1 (see Figure 18). Also, substrate (C) had two additional G vector bases as compared to substrates (A) and (B). The site of cleavage after the A in the AGUC cleavage sequence (see the arrow in Figure 14) of the substrates corresponds to position 320 of the CAT gene using the number system found in the Tn9 sequence (Alton and Vapnak, Nature, 282, 864 (1979)). In Figure 14, the open boxed bases are those which are different from those in the native (-)sTRSV substrate RNA sequence shown in Figure 1.

Catalytic RNAs corresponding to substrate RNAs (A), (B) and (C) were synthesized according to the methods described in Example 2. Their sequences were designed so that they would base pair with the substrate RNAs in both the 3' and 5' regions flanking the AGUC cleavage sequence. In addition, the catalytic RNAs designed to react with substrate RNAs (A) and (B) each contained the vector sequence GA at their 5' terminus, and the catalytic RNA designed to react with substrate RNA (C) contained the vector sequence GGG at its 5' terminus. Otherwise, the catalytic RNAs had the same sequence as the (-)sTRSV catalytic RNA sequence shown in Figure 1.

The various substrate and catalytic RNAs were reacted and the reaction products analyzed as described in Example 3. All reaction conditions were as described in Example 3, except for the following. For substrates (A) and (B), reaction conditions were: substrate RNA concentration = 0.05uM; catalytic RNA concentration = 0.005uM; reaction run at 16°C; and reaction time of 20 minutes. For substrate (C), the reaction conditions were the same as for (A) and (B), except that the reaction time was 40 minutes and temperatures were 20°C, 25°C, 30°C and 37°C.

Cleavage of all of the substrate RNAs occurred when they were mixed with the corresponding catalytic RNAs as is shown in Figure 14, demonstrating that catalytic RNAs according to the invention can be synthesized which cleave specific RNA sequences found within a messenger RNA. In addition, this example demonstrates that cleavage of substrate RNA can be obtained even though the length of the region at the 3' end of the substrate which base pairs with the catalyst (i.e., the portion that forms Helix 1 with the substrate according to the "hairpin" model) is varied. Indeed, when the length of this region was extended to 10 bases in substrate (C), the reaction could then proceed at 37°C, whereas for substrates (A) and (B) having shorter sequences in this region, the reaction would proceed only at 16°C.

### Example 19

An RNA substrate corresponding to part of the sequence of HIV-1, the virus which causes AIDS, was synthesized as described in Example 2. The sequence of this substrate RNA is shown in Figure 15. The arrow in Figure 15 shows the cleavage site which corresponds to position 804 in the sequence of HIV-1 strain SF2CG (Sanchez-Pescador et al., Science, 227, 484 (1985). The sequence shown is found in the region of the viral RNA which specifies the gag protein. The RNA substrate also had a GCG 5' vector sequence. The open boxed bases in Figure 15 are those which are different than those of the native (-)sTRSV substrate sequence shown in Figure 1.

A catalytic RNA was synthesized according to the methods of Example 2. Its sequence was designed so that it would base pair with the substrate RNA in both the 3' and 5' regions flanking the CGUC cleavage sequence. In addition, the catalytic RNA contained the vector sequence GGG at its 5' terminus. Otherwise, the catalytic RNA had the same sequence as the (-)sTRSV catalytic RNA sequence shown in Figure 1.

The catalytic RNA and the substrate RNA were reacted and the reaction products were analyzed as described in Example 3. The reaction conditions were as set forth in Example 3, except that the following temperatures were used: 20°C, 25°C, 30°C and 37°C. Also, the reaction was run for 60 minutes, except for 37^{o}C which was run for 15 minutes, and the substrate RNA concentration was 50nM, and the catalytic RNA concentration was 5nM.

The catalytic RNA cleaved the substrate at the expected position between the "C" and "G" in the CGUC cleavage sequence found in the loop between the two flanking stem regions. Thus, a specific sequence found in the HIV-1 viral RNA that codes for the gag protein can be cleaved. The reaction occurred with an RNA substrate having a 16-base target sequence which was longer than S17 in the region at the 3'end which base pairs with the catalyst (i.e., the portion that forms Helix 1 with the catalytic RNA according to the "hairpin" model). Also, the reaction occurred at physiological temperature of 37°C.

### Example 20

A substrate RNA having a sequence found in the beginning of the coding region for the regulatory protein tat of HIV-1 virus was synthesized as described in Example 2. The substrate sequence is shown in Figure 16. In addition to the HIV-derived sequence, the substrate RNA had a GCG 5' vector sequence. The open boxes are around bases which are different than those of the native (-)sTRSV substrate sequence shown in Figure 1.

A catalytic RNA having a sequence so that it would base pair with the substrate RNA in the two regions flanking the UGUC loop (i.e., the regions that forms Helices 1 and 2 with the catalytic RNA according to the "hairpin" model) was also synthesized as described in Example 2. Otherwise, the catalytic RNAs had the same sequence as the (-)sTRSV catalytic RNA sequence shown in Figure 1, except that it had an additional 5'G vector base.

The substrate RNA and catalytic RNA were reacted and the reaction products were analyzed as described in Example 3. Reaction conditions were: 37°C; reaction times of zero and 15 minutes; the concentration of substrate RNA was 100nM; and the concentration of catalytic RNA was 20mM.

Cleavage occurred as expected between the "U" and the "G" in the UGUC cleavage sequence located between the two stem regions of the substrate. The cleavage site is indicated by the arrow in Figure 16. This is position 5366 in the sequence of HIV clone h9c (Muesing et al., Nature, 313, 450 (1985). These results again confirm that a catalytic RNA designed according to the "hairpin" model can cleave a specific target sequence located in a naturally occurring RNA, in this case a key regulatory region (tat) of the HIV-1 virus which causes AIDS.

### Example 21

Using the methods described in Example 2, a substrate RNA having four non-native bases (UUUU) added to the 3' end of the sequence of the native (-)sTRSV substrate shown in Figure 1 and a corresponding catalytic RNA designed to base-pair with the substrate RNA in the 3' and 5' regions of the substrate flanking the cleavage sequence (i.e., the portions that form Helices 1 and 2 with the catalyst according to the "hairpin" model) were made. Thus, the substrate RNA contained a total of 18 bases, including a four-base extension of the 3' region that base pairs with the catalyst. The substrate RNA also had an additional GCG vector sequence at the 5'end. The catalyst had the same sequence as the (-)sTRSV catalytic RNA sequence shown in Figure 1, except that it had four additional AAAA bases at the 5'end designed to base pair with the added UUUU bases of the substrate and had an additional "G" vector base at the 5' terminus beyond the 18 base recognition site.

The substrate and catalytic RNAs were reacted at standard conditions and the reaction products were analyzed as described in Examples 3 and 5. Catalytic RNA concentration was 0.00033uM, and substrate RNA concentration was 0.1uM (Lane 1), 0.05uM (Lane 2), 0.033uM (Lane 3), 0.025uM (Lane 4), 0.016uM (Lane 5), 0.012uM (Lane 6), 0.006uM (Lane 7), and 0.003uM (Lane 8). The results are shown in Figure 17. Cleavage rates at each concentration of substrate were determined by cutting out the bands, counting radioactivity in a liquid scintillation counter and plotting the data using Michaelis-Menton procedures to calculate Km and kcat (see Example 5).

The data show that an extension of the length of the region of base pairing between the substrate and catalyst (i.e., those regions of the catalyst and substrate that form Helix 1 according to the "hairpin" model) can improve the catalytic properties of the reaction. Cleavage of the 18-base RNA substrate occurred at the expected site, but at an increased rate as compared to the cleavage of S17 by R51. The kcat or turn-over number of the reaction was 7/minute. This means that each molecule of catalytic RNA cleaved 7 molecules of substrate RNA per minute during the reaction. The kcat for S17 cleavage by R51 was 2.1/minute. The Km of the reaction was the same as for S17 cleavage by R51 (30nM). This shows that by optimizing the length of the region of the catalyst that base pairs to the substrate in the 3' region flanking the cleavage sequence (i.e., by optimizing the length of Helix 1 predicted by the "hairpin" model), the catalytic properties of the native reaction can be improved.

### Example 22

A series of catalytic RNAs were prepared using the methods described in Example 2 having certain base substitutions as compared to the native (-)sTRSV catalytic RNA sequence shown in Figure 1. The substitutions, which are illustrated in Figure 19, were the following: (1) At nucleotide 35, G was replaced by C (G35-->C) (count bases from the 5' end of the catalytic RNA sequence shown); (2) A double mutant, having the G at position 35 replaced by C and the C at position 27 replaced by G (G35-->C; C27-->G); (3) At nucleotide 47, G was replaced by C (G47-->C); and (4) A double mutant having the G at position 47 replaced by C and the C at position 17 replaced by G (G47-->C; C17-->G). All catalytic RNAs had an additional "G" vector base at their 5' end.

The various catalytic RNAs were reacted with substrate S17 and the reaction products were analyzed as described in Example 3. The results are shown in Figure 19 where Lanes 1, 3, 5, 7, 9 and 11 are at zero time, and Lanes 2, 4, 6, 8, 10 and 12 are 15 minutes incubation under standard cleavage conditions (see Example 3). The concentration of catalytic RNA was 0.0065uM, and the concentration of substrate RNA was 0.17uM. The temperature was 37°C. The control, lanes 1, 2, 7 and 8, was cleavage of the native substrate RNA S17 by catalytic RNA R51 which has the native (-)sTRSV sequence (see Figure 1).

As shown in Figure 19, the catalytic RNA G35-->C had no catalytic activity (see Lanes 3 and 4 of Figure 19, where this catalytic RNA is designated "mis-match" since the base substitution at position at position 35 results in a loss of base pairing). The double mutant catalytic RNA G35-->C; C27-->G showed restored catalytic activity (see Lanes 5 and 6 of Figure 19 where this catalytic RNA is designated "substitute b.p." since the net effect of the two base substitutions is to create a base pair, but a base pair different than the one found in the native (-)sTRSV catalytic RNA). The catalytic RNA G47-->C was also inactive (see Lanes 9 and 10 of Figure 19), while the double mutant, with the second mutation C17-->G, showed restored activity (see Lanes 11 and 12 of Figure 19).

The results of these mutagenesis studies provide confirmation for the existence of Helices 3 and 4 (see Figure 19) of the "hairpin" catalytic RNA model proposed herein. The results show that when mutagensis was carried out which caused a base pair mismatch in the region of proposed base pairing, the catalytic RNA was inactive. However, when a second mutation was carried out such that the base pair was restored, although the new base pair was different than the original base pair, catalytic activity was restored. Such results are considered evidence for the existence of a helix (Fox and Woese, Nature, 256, 505 (1975). These results also show that a variety of catalytic RNAs having sequences different from the native (-)sTRSV sequence are catalytically active if they are designed so that they follow certain rules derived from the "hairpin" model, such as maintenance of substantial base pairing in regions of predicted helices.

### Example 23

A synthetic "hairpin" autocatalytic cassette was prepared. The RNA sequence of the cassette is shown in Figure 20. Several bases have been added at the 5' end of the catalyst as compared to the native (-)sTRSV sequence shown in Figure 1. The effect is to close the open end of the "hairpin" and to provide a substrate sequence (i.e., a cleavage sequence and upstream and downstream flanking regions) which can base pair with the substrate binding portion of the catalyst.

The cassette was prepared by making an appropriate synthetic DNA template that would yield an RNA with the sequence shown in Figure 20 and using the DNA template to transcribe RNA as described in Example 2, with the following differences. After synthesizing the DNA template, it was inserted into the vector pTZ18R (US Biochemical) into which a new multiple cloning site had been inserted. The new multiple cloning site was a construct containing sites, in 5'->3' order, for cleavage by the following enzymes: BamHI, XhoI, ApaI, SacII, NaeI, StuI, KpnI, MluI. The new multiple cloning site was inserted into vector pTZ18R by cleaving the vector with BamHI and SalI and then ligating the multiple cloning site to the vector using T4 ligase. Located 5' to the inserted multiple cloning site is the T7 RNA polymerase promoter. The vector containing the multiple cloning site was digested with MluI and SalI, and the DNA template coding for the autocatalytic cassette was ligated into the vector using T4 ligase. The resultant vector was then linearized with HindIII, and transcription carried out as described in Example 2. All restriction enzymes and the T4 ligase were obtained from IBI and used according to manufacturer's instructions.

After being transcribed, the cassette spontaneously underwent an intramolecular autocatalytic cleavage at the expected site to give the appropriate 3'F and 5'F products (see Figure 20). Note that the effect of this is to autocatalytically terminate a transcript. For example, the 5'F is in itself a transcript which has been terminated at its 3' end by the autocatalytic reaction. Further note that this termination only leaves five essential bases at the 3' end of this 5'F. These are UGACA which are boxed in Figure 20. Thus, it is possible to very efficiently terminate transcription with this autocatalytic cassette and leave only a very short 3' end to the transcript (i.e., the 5' fragment of the autocatalytic cleavage).

### Example 24

A catalytic RNA was prepared as described in Example 2. Its sequence was identical to that of R51, except that the base at position 217 in the AGAA loop was changed from a G to a C. The AGAA loop of the catalyst is opposite the cleavage sequence of the substrate when the substrate and catalyst are complexed (see Fig. 1) Another catalytic RNA was prepared as described in Example 2. Its sequence was also identical to R51, except that the base at position 216 was changed from A to U.

These catalysts were reacted with the RNA substrate S17 under standard conditions as described in Example 3 at 37°C for 0 and 15 minutes. The control was the reaction of substrate S17 with catalyst R51. The concentration of the three catalytic RNAs was 0.007uM, and the concentration of substrate RNA was 0.7uM.

The reaction products were analyzed as described in Example 3, and the results are shown in Figure 21, where the first lane in each gel is zero time and the second lane is 15 minutes of reaction. As shown in Figure 21, changes in either one of these two bases (G217-->C and A216-->U) in the loop opposite the cleavage sequence of the substrate destroyed the activity of the catalyst. These results indicate that these two bases are likely invariant in the native (-)sTRSV catalytic sequence.

### Example 25

Substrate RNAs identical to S17, but with one of the bases at positions 46, 47 or 48 (i.e., bases GUC of the cleavage sequence) changed to a different base, were prepared as described in Example 2. When these substrates were reacted with the RNA catalyst R51 under standard conditions for 60 minutes as described in Example 3, no cleavage of the substrates occurred. This shows conservation of the GUC sequence of the cleavage sequence of (-)sTRSV RNA. The results of these experiments combined with the results of Examples 13-15 show that the cleavage sequence of (-)sTRSV RNA is NGUC, where N is any base.

### Example 26

The vector prepared in Example 23 (hereinafter referred to as "pHC") containing the "hairpin" autocatalytic cassette was tested for activity in vivo as follows. First, the CAT gene was removed from plasmid pMAMNEO-CAT (purchased from Clontech Inc.) with SmaI and XhoI. It was then ligated using T4 ligase into pHC which had been cut with SmaI and XhoI to produce vector pHC-CAT.

The original vector used in these constructions (pTZ18R; see Example 23) contains an inducible promotor (lacZ) and, as a result of the steps described in Example 23 and immediately above in this example, the CAT gene and the "hairpin" autocatalytic cassette were placed in this inducible region (see Figure 22A). Also, the CAT gene and "hairpin" autocatalytic cassette were linked so that the expected transcript would be as shown in Figure 22A (the "CAT-cassette RNA"). Thus, the RNA sequence coded for by the "hairpin" autocatalytic cassette would be expected to serve as a chain terminator for the CAT transcript by cleaving at the indicated cleavage site (see Figure 22A). The expected 5' fragment of such a cleavage is also shown in Figure 22A ("Cleaved CAT-cassette RNA").

Next, pHC was transfected into Eschericia coli strain JM109 with calcium chloride using standard procedures as described in Maniatis et al., Molecular Cloning (1983). Transformed cells containing pHC-CAT were grown in LB broth at 37^{o}C and then induced with 1mM isopropyl-beta-D-thiogalactoside (IPTG) for one hour to allow expression of the lacZ region, including the CAT gene-cassette RNA transcript.

At the end of this time, RNA was isolated by incubating the cells in 50 mM Tris, pH 8.0, 50mM EDTA, 1 mg/ml lysozyme at room temperature for 10 minutes to lyse the cells. The lysate was made to 0.5% sodium dodecyl sulfate (SDS), and then centrifuged to remove cell debris. Phenol was added to the supernatant at a ratio of 1:1, and the supernatant was centrifuged to remove the precipitate. This procedure was repeated, and the resulting aqueous phase was treated with an equal volume of isopropanol at -20^{o}C for 20 minutes to precipitate the RNA. The precipitate was collected by centrifugation, dried and redissolved in water.

This isolated RNA was electrophoresed on 1.2% agarose gels containing formaldehyde as described in Current Protocols In Molecular Biology (Greene 1989). After electrophoresis, Northern blots were carried out using published methods (GeneScreen Plus, DuPont, July 1985). Two DNA probes were used for blotting the gels. The CAT probe was prepared by primer extension of the CAT gene using the Klenow fragment of DNA polymerase I and dATP labelled with alpha P³². The "hairpin" autocatalytic cassette probe was prepared by kinasing the DNA complement to the entire "hairpin" autocatalytic cassette RNA sequence shown in Figure 20 with dATP labelled with gamma P³². The expected positions of binding of these probes to the CAT-cassette RNA and expected 5' fragment are shown in Figure 22A.

The results of the Northern blot test are shown in figure 22B. As shown there, when the CAT probe was used, the full length CAT-cassette RNA transcript and the expected 5' fragment were detected on the gel, indicating that cleavage had taken place in vivo.

When the "hairpin" autocatalytic cassette probe was used, only the full length CAT-cassette RNA transcript was detected (see Figure 22B). The fact that the 5' fragment did not hybridize with this probe was to be expected, since most of the "hairpin" autocatalytic cassette transcript would be in the 3' fragment after cleavage. The fact that the 3' fragment was not detected by Northern blot analysis is also not surprising. The 5' terminus of the 3' fragment would contain a 5'-OH and not the 5'-ppp which is ordinarily seen in RNA transcripts. Thus, the 3' fragment would be expected to be very labile in vivo and was likely degraded immediately after the autocatalytic cleavage.

The mobilities of the CAT-cassette RNA and the Cleaved CAT-cassette 5' fragment on the gel corresponded exactly to the predicted length of the transcripts. The standards run on the gel were E. coli 16S and 23S RNA.

As a control to determine if cleavage of the target RNA occurred during isolation of the RNA, intact, uncleaved "hairpin" autocatalytic RNA separated on an acrylamide gel as described in Example 23 was isolated from the gel using conditions similar to those described above (i.e., no divalent cation, but in the presence of EDTA and SDS). Only intact, uncleaved RNA was obtained when the catalyst was re-electrophoresed.

As various changes could be made in the above-described products and methods without departing from the scope of the invention, it is intended that all matter contained in the above description or shown in accompanying drawings shall be interpreted as illustrative and shall not be interpreted in a limiting sense.

## Claims

1. A synthetic RNA catalyst capable of cleaving an RNA substrate which contains the sequence:
5'-F₁-CS-F₂-3',
wherein,
CS is a cleavage sequence; and
F₁ and F₂ each is a sequence of bases flanking the cleavage sequence;
the catalyst comprising a substrate binding portion and a "hairpin" portion, the substrate binding portion of the catalyst having the sequence:
3'-F₄-L₁-F₃-5'
wherein,
F₃ is a sequence of bases selected so that F₃ is substantially base paired with F₂ when the catalyst is bound to the substrate;
F₄ is a sequence of base, selected so that F₄ is substantially base paired with F₁ when the catalyst is bound to the substrate;
the sequences of F₃ and F₄ being selected so that each contains an adequate number of bases to achieve sufficient binding of the RNA substrate to the RNA catalyst so that cleavage of the substrate can take place; and
L₁ is a sequence of bases selected so that L₁ does not base pair with CS when the catalyst is bound to the substrate.

2. An RNA catalyst according to Claim 1, the "hairpin" portion of the catalyst having the sequence: 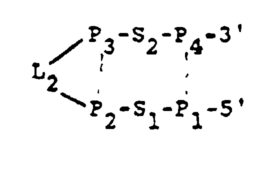 wherein,
P₁ and P₄ each is a sequence of bases, the sequences of P₁ and P₄ being selected so that P₁ and P₄ are substantially base paired;
P₁ is covalently linked to F₄;
S₁ and S₂ each is a sequence of bases, the sequences of S₁ and S₂ being selected so that S₁ and S₂ are substantially unpaired;
P₂ and P₃ each is a sequence of bases, the sequences of P₂ and P₃ being selected so that P₂ and P₃ are substantially base paired; and
L₂ is a sequence of unpaired bases.

3. An RNA catalyst according to Claim 1 or 2 which is capable of cleaving an RNA substrate in which CS has the sequence 5'-NGUC-3', wherein N is any base and the substrate is cleaved by the catalyst between N and G.

4. AN RNA catalyst according to Claim 3 wherein L₁ has the sequence 3'-AAGA-5'.

5. An RNA catalyst according to Claim 1 or 2 wherein F₃ is at least 3 bases in length and F₄ is from 3 to 5 bases in length, and the catalyst cleaves a substrate wherein F₁ and F₂ each is at least 3 bases in length.

6. An RNA catalyst according to Claim 5 wherein F₃ is from 6 to 12 bases in length and F₄ is 4 bases in length, and the catalyst cleaves a substrate wherein F₁ is 4 bases in length and F₂ is from 6 to 12 bases in length.

7. An RNA catalyst according to Claim 2 wherein P₁ and P₄ each is from 3 to 6 bases in length.

8. An RNA catalyst according to Claim 7 wherein P₁ has the sequence 5'-ACCAG-3' and P₄ has the sequence 5'-CUGGUA-3'.

9. An RNA catalyst according to Claim 2 wherein S₁ and S₂ each is from 4 to 7 bases in length.

10. An RNA catalyst according to Claim 9 wherein S₁ has the sequence 5'-AGAA-3' and S₂ has the sequence 5'-AUAUUAC-3'.

11. An RNA catalyst according to Claim 2 wherein P₂ and P₃ each is from 3 to 6 bases in length.

12. An RNA catalyst according to Claim 11 wherein P₂ has the sequence 5'-ACACAC-3' and P₃ has the sequence 5'-GUGGU-3'.

13. An RNA catalyst according to Claim 2 wherein L₂ is at least 3 bases in length.

14. An RNA catalyst according to Claim 13 wherein L₂ has the sequence 5'-GUU-3'.

15. An RNA catalyst according to Claim 2 wherein 5'-S₁-P₂-L₃ has the sequence 5'AGAAACACACGUU-3'.

16. An RNA catalyst according to Claim 1 or 2 which is capable of cleaving an RNA substrate selected from the group consisting of messenger RNA, transfer RNA, ribosomal RNA, viral RNA, nuclear RNA, organellar RNA and other cellular RNA.

17. The catalyst of Claim 16 which is capable of cleaving an RNA substrate selected from the group consisting of HIV-1 virus RNA and tobacco mosaic virus RNA.

18. An RNA catalyst according to Claim 16 which is capable of cleaving messenger RNA coding for chloramphenicol acetyl transferase.

19. An RNA catalyst according to Claim 2 containing the sequence: 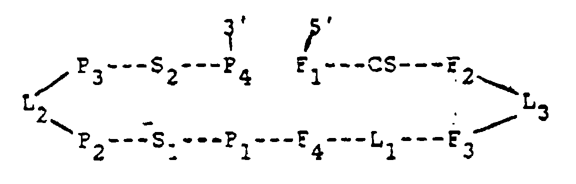 wherein,
F₁, F₂, F₃, F₄, L₁, L₂, S₁, S₂, P₁, P₂, P₃ and P₄ are as defined in Claims 1 and 2; and
L₃ is a sequence of unpaired bases that covalently links the catalyst portion of the molecule with the substrate portion to produce a synthetic autocatalytic RNA catalyst.

20. An RNA catalyst according to Claim 19 wherein CS has the sequence 5'-NGUC-3', wherein N is any base, and the substrate is cleaved by the catalyst between N and G.

21. An RNA catalyst according to Claim 20 wherein L₁ has the sequence 3'-AAGA-5'.

22. An RNA catalyst according to Claim 21 wherein 5'-P₁-S₁-P₂-L₂-P₃-S₂-P₄-3' has the sequence 5'-ACCACAGAAACACACGUUGUGGUAUAUUACCUGGUA-3'.

23. An RNA catalyst according to Claim 22 wherein L₃ had the sequence 3'-CCUCC-5'.

24. A synthetic RNA catalyst which is capable of cleaving an RNA substrate containing the sequence:
5'-F₁-CS-F₂-3',
the catalyst containing the sequence:
5'-F₃ -L₁-F₄-ACCAGAGAAACACACGUUGUGGUAUAUUACCUGGUA-3',
and active variants thereof,
wherein,
CS is a cleavage sequence;
F₁ and F₂ each is a sequence of bases flanking the cleavage sequence:
F₃ is a sequence of bases selected so that F₃ is substantially base paired with F₂ when the catalyst is bound to the substrate;
F₄ is a sequence of bases selected so that F₄ is substantially base paired with F₁ when the catalyst is bound to the substrate;
the sequences of F₃ and F₄ being selected so that each contains an adequate number of bases to achieve sufficient binding of the RNA substrate to the RNA catalyst so that cleavage of the substrate can take place; and
L₁ is a sequence of bases selected so that L₁ does not base pair with CS when the catalyst is bound to the substrate.

25. An RNA catalyst according to Claim 24 wherein F₃ is at least 3 bases in length and F₄ is from 3 to 5 bases in length, and the catalyst cleaver a substrate wherein F₁ and F₂ each is at least 3 bases in length.

26. An RNA catalyst according to Claim 25 wherein F₃ is from 6 to 12 bases in length and F₄ is 4 bases in length, and the catalyst cleaves a substrate wherein F₁ is 4 bases in length and F₂ is from 6 to 12 bases in length.

27. An RNA catalyst according to Claim 24 which is capable of cleaving an RNA substrate in which CS has the sequence 5'-NGUC-3', wherein N is any base and the substrate is cleaved by the catalyst between N and G.

28. AN RNA catalyst according to Claim 27 wherein L₁ has the sequence 3'-AAGA-5'.

29. An RNA catalyst according to Claim 24 which is capable of cleaving an RNA substrate selected from the group consisting of messenger RNA, transfer RNA, ribosomal RNA, viral RNA, nuclear RNA, organellar RNA and other cellular RNA.

30. An RNA catalyst according to Claim 29 which is capable of cleaving an RNA substrate selected from the group consisting of HIV-1 virus RNA and tobacco mosaic virus RNA.

31. An RNA catalyst according to Claim 29 which is capable of cleaving messenger RNA coding for chloramphenicol acetyl transferase.

32. An engineered DNA molecule coding for an RNA catalyst according to claim 1, 2, 19 or 24.

33. A vector comprising a DNA sequence coding for an RNA catalyst according to Claim 1, 2, 19 or 24, the DNA sequence being operatively linked to expression control sequences.

34. The vector of Claim 33 which is capable of self-replication in a host.

35. The vector of Claim 33 wherein the RNA catalyst encoded by the vector is capable of cleaving an RNA substrate selected from the group consisting of messenger RNA, transfer RNA, ribosomal RNA, viral RNA, nuclear RNA, organellar RNA and other cellular RNA.

36. The vector of Claim 35 wherein the RNA catalyst encoded by the vector is capable of cleaving an RNA substrate selected from the group consisting of HIV-1 virus RNA and tobacco mosaic virus RNA.

37. The vector of Claim 35 wherein the RNA catalyst encoded by the vector is capable of cleaving messenger RNA coding for chloramphenicol acetyl transferase.

38. A host cell transformed with a vector according to Claim 33 and which is capable of expressing the RNA substrate.

39. A method of cleaving an RNA substrate which contains the sequence:
5'F₁-CS-F₂-3',
wherein,
CS is a cleavage sequence; and
F₁ and F₂ each is a sequence of bases flanking the cleavage sequence;
the method comprising contacting the substrate with a synthetic RNA catalyst comprising a substrate binding portion and a "hairpin" portion, the substrate binding portion of the catalyst having the sequence:
3'F₄-L₁-F₃-5'
wherein,
F₃ is a sequence of bases selected so that F₃ is substantially base paired with F₂ when the catalyst is bound to the substrate;
F₄ is a sequence of bases selected so that F₄ is substantially base paired with F₁ when the catalyst is bound to the substrate;
the sequences of F₃ and F₄ being selected so that each contains an adequate number of bases to achieve sufficient binding of the RNA substrate to the RNA catalyst so that cleavage of the substrate can take place: and
L₁ is a sequence of bases selected so that L₁ does not base pair with CS when the catalyst is bound to the substrate.

40. The method of Claim 39 wherein the "hairpin" portion of the catalyst has the sequence: 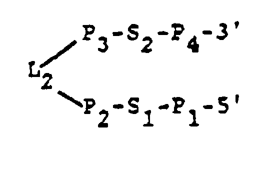 wherein,
P₁ and P₄ each is a sequence of bases, the sequences of P₁ and P₄ being selected so that P₁ and P₄ are substantially base paired;
P₁ is covalently linked to F₄;
S₁ and S₂ each is a sequence of bases, the sequences of S₁ and S₂ being selected so that S₁ and S₂ are substantially unpaired;
P₂ and P₃ each is a sequence of bases, the sequences of P₂ and P₃ being selected so that P₂ and P₃ are substantially base paired; and
L₂ is a sequence of unpaired bases.

41. A method of cleaving an RNA substrate containing the sequence:
5'-F₁-CS-F₂-3',
comprising contacting the substrate with a synthetic RNA catalyst containing the sequence:
5'-F₃-L₁-F₄-ACCAGAGAAACACACGUUGUGGUAUAUUACCUGGUA-3',
and active variants thereof, wherein,
CS is a cleavage sequence;
F₁ and F₂ each is a sequence of bases flanking the cleavage sequence;
F₃ is a sequence of bases selected so that F₃ is substantially base paired with F₂ when the catalyst is bound to the substrate;
F₄ is a sequence of bases selected so that F₄ is substantially base paired with F₁ when the catalyst is bound to the substrate;
the sequences of F₃ and F₄ being selected so that each contains an adequate number of bases to achieve sufficient binding of the RNA substrate to the RNA catalyst so that cleavage of the substrate can take place; and
L₁ is a sequence of bases selected so that L₁ does not base pair with CS when the catalyst is bound to the substrate.

42. The method of Claim 39, 40 or 41 wherein the cleavage occurs under physiological conditions.

43. The method of Claim 42 wherein the cleavage occurs in vivo in a host cell which has been transformed with a vector comprising a DNA sequence coding for the RNA catalyst, the DNA sequence being operatively linked to expression control sequences.
